# EUROPÄISCHE PATENTSCHRIFT

(11) **EP 2 796 938 B1**
(45) Veröffentlichungstag und Bekanntmachung des Hinweises auf die Patenterteilung: **10.06.2015**
(21) Anmeldenummer: 13165409.7
(22) Anmeldetag: 25.04.2013
(51) Int. Cl.: G03H 1/00, G03H 1/04, G03H 1/08, G01B 11/24, G01B 9/02, G03H 1/02

(54) **Vorrichtung zum Erfassen einer 3D-Struktur eines Objekts**
Device for detecting a 3D structure of an object
Dispositif d'enregistrement d'une structure 3D d'un objet

(43) Veröffentlichungstag der Anmeldung: 29.10.2014
(73) Patentinhaber: VOCO GmbH, 27472 Cuxhaven (DE)
(72) Erfinder:
(74) Vertreter: Durm & Partner

(56) Entgegenhaltungen:
- EP-A2- 0 498 575
- WO-A2-2004/094942
- US-A- 4 583 228
- ALEJANDRO CALABUIG ET AL: "Superesolution in digital holographic microscopy", INFORMATION OPTICS (WIO), 2011 10TH EURO-AMERICAN WORKSHOP ON, IEEE, 19. Juni 2011 (2011-06-19), Seiten 1-3, XP031927615, DOI: 10.1109/WIO.2011.5981447 ISBN: 978-1-4577-1227-2
- GIANCARLO PEDRINI ET AL: "Digital holographic interferometry for investigations in biomechanics", PROCEEDINGS OF SPIE, Bd. 5776, 14. Februar 2005 (2005-02-14), Seiten 325-332, XP055076722, ISSN: 0277-786X, DOI: 10.1117/12.611633
- FRANK CHEN ET AL: "Overview of three-dimensional shape measurement using optical methods", OPTICAL ENGINEERING, Bd. 39, Nr. 1, Januar 2000 (2000-01), Seiten 10-22, XP055041587, ISSN: 0091-3286, DOI: 10.1117/1.602438
- J. Schmit ET AL: "Surface Profilers, Multiple Wavelength, and White Light Intereferometry" In: "Optical Shop Testing", 2. November 2006 (2006-11-02), John Wiley & Sons, XP055077154, Seiten 667-752, DOI: 10.1002/9780470135976.ch15, * Abschnitte 15.4-15.9; Seite 695 - Seite 741 *

## Beschreibung

Die vorliegende Erfindung betrifft eine Vorrichtung zum Erfassen einer dreidimensionalen Struktur eines Objekts umfassend, einen ersten Laser-Emitter, der Laserstrahlung mit einer ersten Wellenlänge erzeugt, einen zweiten Laser-Emitter, der Laserstrahlung mit einer zweiten Wellenlänge erzeugt, wobei sich die erste Wellenlänge von der zweiten Wellenlänge unterscheidet, optische Einrichtungen, von denen wenigstens eine ein Strahlteiler ist, der die Laserstrahlung der Laser-Emitter jeweils in eine Referenzstrahlung und eine Beleuchtungsstrahlung aufteilt. Die Beleuchtungsstrahlung trifft auf das zu vermessende Objekt, wird von dem Objekt als Objektstrahlung reflektiert und interferiert mit der Referenzstrahlung. Ein Detektor der Vorrichtung nimmt die daraus entstehenden Interferenzmuster auf. Die Vorrichtung umfasst eine Messeinrichtung, welche es erlaubt, den zeitlichen Verlauf der Wellenlängen der Laserstrahlung der Laser-Emitter zu messen.

Vorrichtungen zum Erfassen von 3D-Strukturen werden beispielsweise in der Industrie zur Qualitätssicherung eingesetzt. Bearbeitete Oberflächen können so auf ihre Güte hin, zum Beispiel auf Einhaltung von Maßhaltigkeit und/oder auf Einhaltung einer vorgegebenen Rauheit, untersucht werden. Darüber hinaus ist es mit solchen Vorrichtungen möglich, ganze Objekte digital zu erfassen und dreidimensional abzubilden.

Aus der US 6,809,845 B1 ist eine solche Vorrichtung bekannt. Diese arbeitet nach dem Prinzip der Holographie. Mittels zweier Laser werden zwei Laserstrahlen mit unterschiedlicher Wellenlänge erzeugt. Jeder Laserstrahl wird in einen Objektstrahl, der auf ein zu vermessendes Objekt trifft, und einen Referenzstrahl aufgeteilt. Der vom Objekt reflektierte Objektstrahl und der zugehörige Referenzstrahl einer Wellenlänge werden zusammengeführt und interferieren miteinander, wobei die Phasenbeziehungen zwischen den beiden Strahlen aufgenommen werden. Mithilfe der Differenz zwischen den Phasenbeziehungen, erzeugt durch den Laserstrahl einer ersten Wellenlänge, und den Phasenbeziehungen, erzeugt durch den Laserstrahl einer zweiten Wellenlänge, kann ein dreidimensionales Modell der reflektierten Oberfläche des Objekts erstellt werden. Zusätzlich zu den Lasern, einem Nd:YAG-Laser (Neodym-dotierter Yttrium-Aluminium-Granat-Laser) und einem HeNe-Laser (Helium-Neon-Laser), umfasst die Vorrichtung mehrere Spiegel, Strahlteiler, Filter und Blenden. Der benötigte Platzbedarf der Vorrichtung ist groß.

Die US 8,068,235 B1 offenbart ebenfalls eine Vorrichtung zur dreidimensionalen Erfassung von Oberflächenstrukturen. Zwei Laserlichtquellen erzeugen einen Laserstrahl. Teile dieser Laserstrahlen werden zu einem Verbundstrahl zusammengeführt, der auf das zu vermessende Objekt trifft. Er wird anschließend vom Objekt reflektiert und von einer Kamera detektiert. Die übrigen Teile der emittierten Laserstrahlen treffen als Referenzstrahlen mit unterschiedlichen Einfallswinkeln auf die Kamera auf. Indem die Laserstrahlen teilweise über Strahlteiler zusammengeführt werden, weist die Vorrichtung einen erhöhten Platzbedarf auf.

Bei beiden Patentschriften trifft das Licht von zwei oder mehreren Wellenlängen auf das Objekt unter identischen Winkeln auf. Das verursacht sogenanntes Specklerauschen, weil durch die Wellennatur des Lichtes im rückgestreuten Licht eines etwas rauen Oberflächen-Objektes Zonen sogenannter konstruktiver (hell) und destruktiver (dunkel) Interferenz entstehen. Dadurch sieht das Objekt für den Betrachter "granular" aus, was als Speckle bzw. Specklerauschen bezeichnet wird.

Aus der EP 0 498 575 A2 ist ein Interferometer mit zwei Laserquelle bekannt, die jeweils Laserstrahlung mit einer unterschiedlichen Wellenlänge aussenden. Die Laserstrahlung wird in Referenz- und Beleuchtungsstrahlung aufgeteilt, wobei die Beleuchtungsstrahlung an einem Objekt reflektiert und mit der Referenzstrahlung auf einem Detektor interferiert. Das Interferometer weist eine Messeinrichtung mit einem Fabry Perot Etalon auf. Mithilfe des Fabry Perot Etalons werden die Wellenlängen der ausgesandten Laserstrahlung gemessen und die Laserquellen derart gesteuert, dass Langzeit-Wellendrifts infolge thermischer Effekte vermieden werden. Die Steuerung der Laserquellen in Abhängigkeit von den gemessenen Wellenlängen ist sehr aufwendig.

Die US 4583228 A offenbart eine Laserquelle, die Laserstrahlug mit einer bestimmten Wellenlänge aussendet. Mithilfe eines Fabry Perot Interferometers wird die Wellenlänge der ausgesendeten Laserstrahlung gemessen. Zwei Rückführkreise steuern die Laserquelle basierend auf der Messung des Fabry Perot Interferometers derart, dass die Laserquelle Laserstrahlung mit einer stabilen Wellenlänge aussendet.

Weitere Vorrichtungen, die nach dem Prinzip der digitalen Holografe arbeiten sind aus der WO 2004/094942 A2, dem Dokument Superesolution in digital holographic microscopy, Alejandro Calabuig et al, dem Dokument Digital holographic interferometry for investigations in biomechanics, Giancarlo Pedrini et al, dem Dokument Overview of three-dimensional shape measurement u-sing optical methods, Frank Chen et al, und dem Dokument Surface profilers, Multiple wavelength and white light interferometry, J. Schmit et al, bekannt.

Aus oben genannten Gründen ergibt sich die Aufgabe, eine kompakte Vorrichtung zum Erfassen einer 3D-Struktur eines Objekts, sowie zur Minimierung des Specklerauschens bereitzustellen, die die beschriebenen Nachteile vermeidet.

Bei der Lösung dieser Aufgabe wird von einer Vorrichtung gemäß dem Oberbegriff des Anspruchs 1 ausgegangen. Gelöst wird die Aufgabe durch die im Kennzeichen des Anspruchs 1 angegebenen konstruktiven Merkmale. Weitere Ausführungsformen finden sich in den abhängigen Ansprüchen.

Die Vorrichtung umfasst eine Messeinrichtung, die die beiden Wellenlängen der Laserstrahlung der Laser-Emitter misst. Die Messeinrichtung beeinflusst die Aufnahmen der Interferenzmuster durch den Detektor. Störeinflüsse, wie beispielsweise Temperaturveränderungen können dazu führen, dass die Laser-Emitter Strahlung mit schwankenden Wellenlängen erzeugen. Mithilfe der Messeinrichtung ist gewährleistet, dass die Wellenlängen der von den Laser-Emittern erzeugten Laserstrahlung bei der Auswertung der Interferenzmuster und deren zeitlicher Verlauf exakt bekannt sind.

Die Messeinrichtung misst den zeitlichen Verlauf der Wellenlängen der Laserstrahlung der Laser-Emitter, wobei eine mit der Messeinrichtung verbundene Steuereinrichtung den Detektor im Falle im Wesentlichen konstanter Wellenlängen ansteuert und eine Aufnahme der Interferenzmuster auslöst.

Bei einer Aufnahme der Interferenzmuster durch den Detektor ist es vorteilhaft, wenn der zeitliche Verlauf der Wellenlängen im Wesentlichen konstant ist. Im Rahmen der vorliegenden Erfindung werden unter dem Begriff im Wesentlichen konstante Wellenlängen Wellenlängenschwankungen im Bereich des 10⁻⁶ und 10⁻⁷ fachen der eingestrahlten Wellenlänge, also im Bereich von ungefähr 0,1 - 1 pm verstanden. Die Interferenzmuster werden mit einer Belichtungszeit von bevorzugt 10 - 100 µs aufgenommen. Durch die kurze Belichtungszeit ist gewährleistet, dass auch bei moderat bewegten Objekten die phasensensitive Aufnahme nicht verwackelt.

Es ist auch denkbar, dass die Vorrichtung eine Regeleinrichtung aufweist, die in Abhängigkeit der Messergebnisse der Messeinrichtung die Laser-Emitter derart regelt, dass die Wellenlängen der emittierten Laserstrahlung im Wesentlichen konstant sind.

Die Laser-Emitter sind derart angeordnet, dass die Beleuchtungsstrahlung des ersten Laser-Emitters und die Beleuchtungsstrahlung des zweiten Laser-Emitters mit unterschiedlichen Einfallswinkeln auf das Objekt treffen. Bevorzugt trifft die Beleuchtungsstrahlung der jeweiligen Laser-Emitter unter einem relativ kleinen Einfallswinkel auf das Objekt auf. Beispielsweise liegt bei zwei Laser-Emittern der typische Einfallswinkel bei ca. 0,11°. Im Rahmen der Erfindung wurde erkannt, dass in vorteilhafter Weise bei der Verwendung von mehr als zwei Laser-Emittern - also z.B. 8 - die Beleuchtungsstrahlung mit Einfallswinkeln von höchstens 1°, bevorzugt von ca. 0,8°, auf das Objekt treffen. Größere Einfallswinkel der Beleuchtungsstrahlung sind ebenfalls denkbar.

Bevorzugt emittieren die Laser-Emitter Laserstrahlung mit einer Gesamtleistung von mindestens 100 mW, wobei die Gesamtleistung stark von der Belichtungszeit abhängt.

Unter dem Begriff Beleuchtungsstrahlung wird im Rahmen der Erfindung der Teil der emittierten Laserstrahlung verstanden, der auf das zu vermessende Objekt trifft. Mit dem Begriff Objektstrahlung wird die an dem Objekt reflektierte Beleuchtungsstrahlung bezeichnet. Die Referenzstrahlung ist der Teil der emittierten Laserstrahlung, die als Referenz unverändert mit der Objektstrahlung interferiert und auf den Detektor trifft.

Im Rahmen der vorliegenden Erfindung wurde erkannt, dass die erfindungsgemäße Vorrichtung auch im Bereich des menschlichen Mundes einsetzbar ist. Insbesondere ist es denkbar, die Vorrichtung zum Erfassen von Oberflächen und Objekten im Mundinnenbereich, beispielsweise als Dental-Scanner, einzusetzen. Um diesen Anforderungen gerecht zu werden, weist die Vorrichtung bevorzugt entsprechende Dimensionen auf, die ein zumindest teilweises Einführen der Vorrichtung in den Mundinnenbereich ermöglichen.

Es ist zweckmäßig, die Messeinrichtung als Fabry Perot Interferometer auszubilden. Ein Fabry Perot Interferometer ist im Allgemeinen aus dem Stand der Technik bekannt. Im vorliegenden Fall umfasst ein Resonator des Fabry Perot Interferometers zwei Glasplatten, auf denen jeweils Spiegel aufgedampft sind. In Abhängigkeit von den Resonanzbedingungen des Resonators wird die Laserstrahlung mit bestimmten Wellenlängen durch die aufgedampften Spiegel transmittiert und im vorliegenden Fall durch ein Detektor-Array detektiert. Mithilfe des Fabry Perot Interferometers ist es möglich, die Wellenlängen, mit denen die jeweiligen Laser-Emitter Laserstrahlung aussenden, in einem bestimmten Wellenlängenbereich exakt zu messen. Das Fabry-Perot Interferometer setzt eine gewisse Grundstabilität der Wellenlängen der von den Laser-Emittern ausgesendeten Laserstrahlung voraus. Heutige Laser-Emitter sind aber in der Lage, eine solche Grundstabilität bereitzustellen.

Bevorzugt sind der erste Laser-Emitter und der zweite Laser-Emitter auf einem gemeinsamen Emitter-Chip voneinander beabstandet angeordnet. Beispielsweise sind die Laser-Emitter als Multilaserdioden ausgebildet. Bevorzugt sind die einzelnen Laser-Emitter mit einem Abstand von höchstens 0.5 mm, besonders bevorzugt mit einem Abstand von höchstens 0.2 mm, insbesondere bevorzugt mit einem Abstand von höchstens 0.1 mm zueinander angeordnet.

Durch solch geringe Abstände sind multiple Emitter auf sehr engem Bauraum möglich. Mehrere Emitter bilden eine Wellenlängen-Gruppe, mittels der 3D Oberflächeninformationen eines Objekts und Informationen über die Tiefe einzelner Objektpunkte auf dem Objekt (Oberflächenpunkte am Objekt) erfasst werden. Es können auch zwei oder mehr Wellenlängen-Gruppen auf dem gleichen Chip anordnet sein, um eine Speckle-Reduktion durchzuführen.

Vorteilhaft weist die Vorrichtung höchstens zweiunddreißig Laser-Emitter auf, wobei bevorzugt jeweils höchstens sechzehn Laser-Emitter auf einem Emitter-Chip angeordnet sind. Beispielsweise senden alle auf einem Emitter-Chip angeordneten sechszehn Laser-Emitter jeweils Laserstrahlung mit einer anderen Wellenlänge aus. Alle sechzehn Laser-Emitter bilden eine WellenlängenGruppe, der eine zentrale Wellenlänge zugeordnet werden kann. Unter der zentralen Wellenlänge wird im Rahmen der vorliegenden Erfindung der Mittelwert aller Wellenlängen verstanden, die von Laser-Emittern einer Wellenlängen-Gruppe emittiert werden.

Es ist aber auch denkbar, dass sich sechzehn Laser-Emitter in vier Reihen zu je vier Laser-Emittern nebeneinander auf einem Emitter-Chip befinden. Jede Reihe mit jeweils vier Laser-Emittern bildet eine Wellenlängen-Gruppe, der eine zentrale Wellenlänge zugeordnet werden kann. Jeder der Laser-Emitter einer Wellenlängen-Gruppe emittiert Laserstrahlung mit einer unterschiedlichen Wellenlänge. Die einzelnen Reihen, d.h. die unterschiedlichen Wellenlängen-Gruppen, stimmen bevorzugt in ihren zentralen Wellenlängen überein.

Eine zentrale Wellenlänge kann bevorzugt zwischen 750 und 850 nm, insbesondere ca. 800 nm betragen. Auch zentrale Wellenlängen um 950 nm oder gar 1300 nm sind möglich. Zentrale Wellenlängen im sichtbaren Spektralbereich sind ebenfalls denkbar, wenngleich dies technologisch anspruchsvoll zu realisieren ist.

Es sind weitere Konfigurationen denkbar, wobei bevorzugt ein, zwei oder vier Wellenlängen-Gruppen auf einem Emitter-Chip angeordnet sind. Die Anzahl der Laser-Emitter in einer Wellenlängen-Gruppe kann variieren und ist nicht auf vier, sechzehn oder zweiunddreißig Laser-Emitter festgelegt. Auch ist die Anordnung der Emitter in Bezug auf die einzelnen Wellenlängen nicht zwingend monoton steigend oder fallend, sondern kann auch "zufällig" sein. Ebenso kann die Anzahl an Gruppen auf einem Emitter-Chip von der beschriebenen Anzahl abweichen.

Es ist vorteilhaft, mehrere Wellenlängen-Gruppen auf einem Emitter-Chip nebeneinander anzuordnen, da dann eine Speckle-Reduktion erfolgen kann. Durch Reflexion der Laserstrahlung an einer unebenen Oberfläche eines Objekts entsteht ein Speckle-Muster, dessen Ausgestaltung stark von dem Einfallswinkel der Beleuchtungsstrahlung auf das Objekt abhängig ist und eine Objekterkennung erschwert. Werden mehrere Wellenlängen-Gruppen nebeneinander auf einem Emitter-Chip angeordnet, wird ein größeres Winkelspektrum hinsichtlich der Einfallswinkel der Beleuchtungsstrahlung erzeugt. Für jede Wellenlängen-Gruppe wird dann die Tiefeninformation derselben beleuchteten Objektpunkte unter unterschiedlichem mittleren Einstrahlwinkel erfasst. Unter dem mittleren Einstrahlwinkel wird der über die Einstrahlwinkel einer Wellenlängen-Gruppe gemittelte Einstrahlwinkel verstanden. Eine Mittelwertbildung der Tiefeninformationen über die Wellenlängen-Gruppen bewirkt eine Reduktion des Einflusses der Speckle-Muster.

Soll eine Speckle-Reduktion erfolgen, ist es vorteilhaft, wenn die unterschiedlichen Wellenlängen-Gruppen in ihren zentralen Wellenlängen übereinstimmen. Dadurch wird der Aufwand bei der späteren Auswertung der Interferenzmuster gering gehalten. Es ist aber auch denkbar, eine Speckle-Reduktion durchzuführen, wenn die einzelnen Wellenlängen-Gruppen Laserstrahlung mit unterschiedlichen zentralen Wellenlängen aussenden. Unterscheiden sich die zentralen Wellenlängen der Wellenlängen-Gruppen voneinander, liegen sie eng aneinander, d.h. die zentralen Wellenlägen unterscheiden sich um ca. 50 - 100 nm.

Es versteht sich, dass auch zwei oder mehr Emitter-Chips mit jeweils einer oder mehreren Wellenlängen-Gruppen verwendet werden können, wobei die zentralen Wellenlängen übereinstimmen oder sich unterscheiden können.

Es wird ausdrücklich darauf hingewiesen, dass die vorliegende Erfindung nicht auf eine Anzahl von zweiunddreißig Laser-Emittern limitiert ist. Die Konfiguration mit insgesamt zweiunddreißig Laser-Emittern, aufgeteilt auf zwei Emitter-Chips, ist lediglich eine bevorzugte Ausführungsform. Es ist denkbar weitaus mehr als insgesamt zweiunddreißig Laser-Emittern, vorzugsweise aufgeteilt auf mehrere Emitter-Chips / Wellenlängengruppen, zu verwenden. Je höher die Anzahl der Wellenlängengruppen ist desto besser kann entstehendes Signalrauschen bei der Auswertung der Interferenzmuster unterdrückt werden. Die Anzahl der verwendeten Laser-Emitter und Wellenlängengruppen wird nur durch die Größe / Pixeldichte des Detektors bzw. das ausgewählte Beleuchtungsfeld auf dem Objekt und die vorhandene Rechenleistung bei der Auswertung der Interferenzmuster limitiert.

Eine der optischen Einrichtungen ist ausgebildet, die Referenzstrahlung derart zu reflektieren, dass die Referenzstrahlung der einzelnen Laser-Emitter mit unterschiedlichen Einfallswinkeln auf den Detektor treffen. Bevorzugt treffen Referenzstrahlung und Objektstrahlung einer Wellenlänge mit unterschiedlichen Einfallswinkeln auf den Detektor. Beispielsweise trifft die Objektstrahlung, die an einem Objektpunkt des zu untersuchenden Objekts reflektiert wurde, unabhängig von ihrer Wellenlänge unter dem gleichen Einfallswinkel auf einem Detektor auf. Es wird in diesem Zusammenhang von einer Mindestrauheit der Oberflächenstruktur des Objekts ausgegangen. Im Falle eines ideal glatten Objekts ergeben sich abweichende Verhältnisse für die Objektstrahlung.

Der Strahlverlauf von Referenz- und Beleuchtungsstrahlung ist teilweise überlagert, so dass Referenzstrahlung und Objektstrahlung einer Wellenlänge, d.h. von demselben Laser-Emitter, miteinander interferieren. Es bildet sich ein Interferenzmuster pro emittierter Wellenlänge, so dass aus allen wellenlängenabhängigen Interferenzmustern, beispielsweise in einer Verarbeitungseinrichtung, mittels der Fourier-Transformation oder der Fresnel-Transformation die Position des Objektpunkts auf der Oberfläche des Objekts und die Position des Objektpunktes in der Tiefe bestimmt wird.

In einer optionalen Ausführungsform ist wenigstens eine der optischen Einrichtungen ein Hologramm. Das Hologramm lenkt die Beleuchtungsstrahlung derart ab, dass sie als Beleuchtungsstreifen auf das Objekt trifft. Beispielsweise ist das Hologramm als Mikro-Hologramm ausgeführt. Soll ein Objekt erfasst werden, wird dieses während einer Relativbewegung zwischen Vorrichtung und Objekt entlang einer Scanrichtung abgetastet. Bevorzugt ist der Beleuchtungsstreifen rechteckig ausgebildet, wobei die kurzen Seiten des Rechtecks parallel zur Scanrichtung verlaufen und die langen Seiten quer zur Scanrichtung ausgerichtet sind. Das Mikro-Hologramm verändert die numerische Apertur der Beleuchtungsstrahlung derart, dass die numerische Apertur entlang der langen Seiten des rechteckförmigen Beleuchtungsstreifens größer ist als die numerische Apertur entlang der kurzen Seiten des rechteckförmigen Beleuchtungsstreifens.

Soll beispielweise eine Schweißnaht erfasst werden, gibt der Verlauf der Schweißnaht die Scanrichtung vor. Da die langen Seiten des rechteckförmigen Beleuchtungsstreifen quer zur Scanrichtung verlaufen, kann somit die gesamte Breite der Schweißnaht mithilfe des Beleuchtungsstreifens erfasst werden.

Da kurze Belichtungszeiten bei der Aufnahme der Interferenzmuster verwendet werden, überlappen sich teilweise vom Detektor aufgenommene Bereiche der Schweißnaht. Dies ermöglicht es, Höhenunterschiede, beispielsweise durch Bewegung des Objekts relativ zu dem Detektor oder durch mechanische Fehler der Vorrichtung bei der Detektion, auszugleichen.

Es ist aber auch denkbar, dass das Hologramm die Beleuchtungsstrahlung aufteilt und derart ablenkt, dass zwei Beleuchtungsstreifen auf das Objekt treffen. Vorteilhafterweise sind beide Beleuchtungsstreifen entlang der Scanrichtung (kurze Seite) angeordnet. Dies ermöglicht einen Betrieb der erfindungsgemäßen Vorrichtung als Handscanner. Indem zwei Beleuchtungs-Streifen verwendet werden, können eventuelle Relativbewegungen insbesondere rotatorische Relativbewegungen der Vorrichtung relativ zu dem Objekt erkannt und bei der Auswertung berücksichtigt werden. Es versteht sich, dass das Hologramm die Beleuchtungsstrahlung auch derart aufteilen und ablenken kann, dass mehr als zwei Beleuchtungsstreifen auf das Objekt treffen.

Vorteilhafterweise ist wenigstens eine der optischen Einrichtungen ein mikro-optisches Array. Das mikro-optische Array ist eine Kombination von verschiedenen optischen Bauteilen, wie beispielsweise Linsen, Strahlteilern, Zirkulatoren und/oder Hologrammen, die sehr kompakt angeordnet sind. Das mikro-optische Array wird bevorzugt im Strahlengang direkt hinter den Laser-Emittern angeordnet.

Es ist zweckmäßig, dass das mikro-optische Array den Strahlteiler umfasst, der die Laserstrahlung der Laser-Emitter in die Referenzstrahlung und die Beleuchtungsstrahlung aufteilt. Bevorzugt verlaufen die Referenz- und Beleuchtungsstrahlung zumindest teilweise überlappend. Somit ist ein besonders kompakter Aufbau der Optik der Vorrichtung möglich.

Bevorzugt umfasst das mikro-optische Array einen Polarisator und/oder Zirkulator, um die Beleuchtungsstrahlung und/oder die Referenzstrahlung zu polarisieren. Bevorzugt wird die Referenzstrahlung relativ zur Beleuchtungsstrahlung so polarisiert, dass die Polarisationsebene mithilfe eines Polarisators und/oder Zirkulators um 90 Grad gedreht wird. Die Polarisation der Beleuchtungsstrahlung wird hingegen nicht beeinflusst. Dies ist besonders vorteilhaft, wenn nach dem mikro-optischen Array ein polarisierender Strahlteiler im Strahlengang angeordnet ist, der beispielsweise Laserstrahlung einer ersten Polarisation, insbesondere die Referenzstrahlung, durchlässt und Laserstrahlung mit einer zweiten Polarisation, insbesondere die Beleuchtungsstrahlung, reflektiert. Es ist ebenfalls denkbar, die Beleuchtungsstrahlung mithilfe des Polarisators und/oder des Zirkulators um 90° zu drehen und die Referenzstrahlung unbeeinflusst zu lassen.

Auch kann das mikro-optische Array ein Hologramm umfassen. Dadurch werden die Anforderungen an die Genauigkeit von im Strahlengang befindlicher Linsen reduziert. Dies verringert die bei der Herstellung der Vorrichtung entstehenden Kosten.

Bevorzugt bewirkt das Hologramm im mikro-optischen Array auch, dass die Referenz- und Beleuchtungsstrahlung jeweils mit einer unterschiedlichen numerischen Apertur das mikro-optische Array verlässt. Beispielsweise weist die Referenzstrahlung eine hohe numerische Apertur auf, während die Beleuchtungsstrahlung das mikro-optische Array zumindest entlang der kurzen Seiten des rechteckförmigen Beleuchtungsstreifens mit einer sehr geringeren numerischen Apertur verlässt. Darüber hinaus ist es mithilfe eines Hologramms auf einfache Weise möglich, mehrere Beleuchtungsstreifen beispielsweise zwei Beleuchtungsstreifen zu erzeugen.

Optional ist eine der optischen Einrichtungen als chromatisch dispersive Linse ausgebildet. Bevorzugt wird die chromatisch dispersive Linse im Strahlengang der Objektstrahlung angeordnet, d.h. in Strahlrichtung nach der Reflexion der Beleuchtungsstrahlung am Objekt. Dadurch lässt sich der Aufwand der Fourier- oder Fresnel-Transformation bei der Auswertung der Interferenzmuster verringern.

Eine weitere Ausführungsform der Erfindung ist dadurch gekennzeichnet, dass die Vorrichtung zwei Emitter-Chips und zwei Detektoren aufweist, wobei die Laserstrahlung mit einer ersten zentralen Wellenlänge der auf dem einen Emitter-Chip angeordneten Laser-Emitter auf den einen Detektor und die Laserstrahlung einer zweiten zentralen Wellenlänge der auf dem anderen Emitter-Chip angeordneten Laser-Emitter auf den anderen Detektor trifft. Werden z.B. zwei Emitter-Chips mit unterschiedlichen zentralen Wellenlängen verwendet, können die einzelnen Laserstrahlungen beispielsweise mittels Strahlteiler, die die Laserstrahlung abhängig von ihren zentralen Wellenlängen durchlassen oder reflektieren, so aufgeteilt werden, dass Laserstrahlung mit der ersten zentralen Wellenlänge auf einen ersten Detektor und die Laserstrahlung mit einer zweiten zentralen Wellenlänge auf einen zweiten Detektor fällt.

Vorteilhafterweise weist die Vorrichtung einen Messeinheit zur Messung der Dicke einer auf dem Objekt angeordneten Schicht auf. Somit kann zusätzlich zur Oberfläche des Objekts auch die Dicke einer evtl. auf dem Objekt vorhandenen Schicht erfasst werden. Wird die Vorrichtung als Dentalscanner eingesetzt kann so die Dicke von Zahnfleisch auf einem Zahn gemessen werden. Beispielsweise kann eine solche Schichtdickenmessung mit der erfindungsgemäßen Vorrichtung ohne zusätzliche Bauteile umgesetzt werden. Bevorzugt ist die Messeinheit als Teil der Verarbeitungseinrichtung ausgebildet. Mithilfe der Phaseninformationen aus den Interferenzmustern mehrerer Wellenlängen können nicht nur eine sondern auch mehrere Oberflächen, beispielsweise zwei oder drei Oberfläche, erfasst werden. Grundsätzlich gilt, je mehr Wellenlängen in der Wellenlängen-Gruppe, desto sicherer werden eine oder mehrere zusätzliche Oberflächen erkannt. Bevorzugt werden zwei Oberflächen mittels der Vorrichtung erfasst und zueinander in Bezug gesetzt. Als erste Oberfläche wird die äußere Oberfläche einer auf dem Objekt angeordneten Schicht und als zweite Oberfläche die Oberfläche des Objekts, auf der sich die zu vermessende Schicht befindet, erfasst. Die Abstandsdifferenz der beiden Oberflächen liefert unter Kenntnis des Brechungsindex die Schichtdicke, beispielsweise die Dicke des Zahnfleischs.

Eine ebenfalls bevorzugte Ausführungsform ist dadurch gekennzeichnet, dass die Messeinheit einen Weißlicht-Punktsensor umfasst, der nach dem Prinzip der frequenz-scannenden Interferometrie arbeitet. Das Prinzip der frequenz-scannenden Interferometrie ist aus dem Stand der Technik bekannt. Alternativ sind auch andere Verfahren für diese Messeinheit denkbar. Bevorzugt weist der Weißlicht-Punktsensor eine Lichtquelle auf, die Licht mit einem breiten Spektrum, bevorzugt um eine zentrale Wellenlänge von 1300 nm und einer Breite des Spektrums zwischen 10 und 100 nm erzeugt. Mithilfe des Weißlicht-Punktsensors mit einer zentralen Wellenlänge von 1300 nm lassen sich besonders streuende Schichten, beispielsweise das Zahnfleisch direkt vermessen. Nach Vermessen der Zahnfleischdicke wird diese dann mit der Oberfläche des Zahnfleischs, die ohne Messeinheit nur mit der erfindungsgemäßen Vorrichtung erfasst wurde, in Bezug gesetzt. Dadurch ist eine Ermittlung der 3D Zahnoberfläche unterhalb des Zahnfleisches möglich.

Auch kann die Messeinheit ausgebildet sein, basierend auf dem Prinzip der Ellipsometrie die Dicke der auf dem Objekt angeordneten Schicht zu ermitteln. Auch das Prinzip der Ellipsometrie ist aus dem Stand der Technik bekannt. Mithilfe des ellipsometrischen Ansatzes können sehr dünne Schichten, d.h. zwischen etwa 0,01 µm und 1 µm vermessen werden. Hierzu wird erfindungsgemäß die Tatsache genutzt, dass verschiedene Emitter auf unterschiedlichen lateralen Positionen auf dem Multi-Emitter Chip sitzen und so Licht bei unterschiedlichen Winkeln auf das Objekt einstrahlen lassen können. Zusammen mit fest eingestellten Polarisatoren an einzelnen Emittern in der Beleuchtungsstrahlung und/oder fest eingestellten Analysatoren (Polarisatoren) an unterschiedlichen Stellen in der Objektstrahlung lässt sich aus der Summe aller Informationen eine dünne Beschichtungsstärke ermitteln. Gleichzeitig ist die komplette 3D Oberflächeninformation des Objekts nach oben beschriebener Vorgehensweise verfügbar.

Erfindungsgemäß lässt sich eine Vorschalt-Optik so ausgestalten, dass Licht auch unter recht flachem Winkel auf das Objekt einstrahlt, was die Sensitivität für dünne Beschichtungen bei dem ellipsometrischen Ansatz bekanntermaßen deutlich erhöht.

Die Erfindung wird nachfolgend anhand von in den Figuren dargestellten bevorzugten Ausführungsformen erläutert. Die darin dargestellten Besonderheiten können einzeln oder in Kombination verwendet werden, um bevorzugte Ausgestaltungen der Erfindung zu schaffen. Die beschriebenen Ausführungsformen stellen keine Einschränkung der Allgemeinheit des in den Ansprüchen definierten Gegenstands dar. Es zeigen:
- Figur 1a: eine erfindungsgemäße Vorrichtung gemäß einer ersten Ausführungsform in einem Mach-Zehnder Aufbau, schematisch dargestellt,
- Figur 1b: die erfindungsgemäße Vorrichtung gemäß einer zweiten Ausführungsform mit einer Messeinheit, die basierend auf dem Prinzip der Ellipsometrie arbeitet, in einer schematischen Darstellung,
- Figur 2: die erfindungsgemäße Vorrichtung gemäß einer dritten Ausführungsform in einem Michelson-Aufbau, schematisch dargestellt,
- Figur 3: ein mikro-optisches Array gemäß einer ersten Ausführungsform mit einem Strahlteiler in einer Seitenansicht,
- Figur 4: das mikro-optische Array gemäß einer zweiten Ausführungsform mit einem Hologramm in einer Seitenansicht,
- Figur 5: die erfindungsgemäße Vorrichtung gemäß einer vierten Ausführungsform mit einem Hologramm zur Erzeugung mehrerer Beleuchtungsstreifen in einer schematischen Darstellung,
- Figur 6: die erfindungsgemäße Vorrichtung gemäß einer fünften Ausführungsform mit zwei Detektoren in einer schematischen Darstellung,
- Figur 7: die erfindungsgemäße Vorrichtung gemäß einer sechsten Ausführungsform, ausgebildet als Innenbohrungs-Scanner, in einer schematischen Darstellung,
- Figur 8: die erfindungsgemäße Vorrichtung gemäß einer siebten Ausführungsform mit einem Hologramm in einem mikro-optischen Array in einer schematischen Darstellung und
- Figur 9: die erfindungsgemäße Vorrichtung gemäß einer achten Ausführungsform mit einer Messeinheit, die nach dem Prinzip der Weißlicht-Interferometrie arbeitet, in einer schematischen Darstellung.

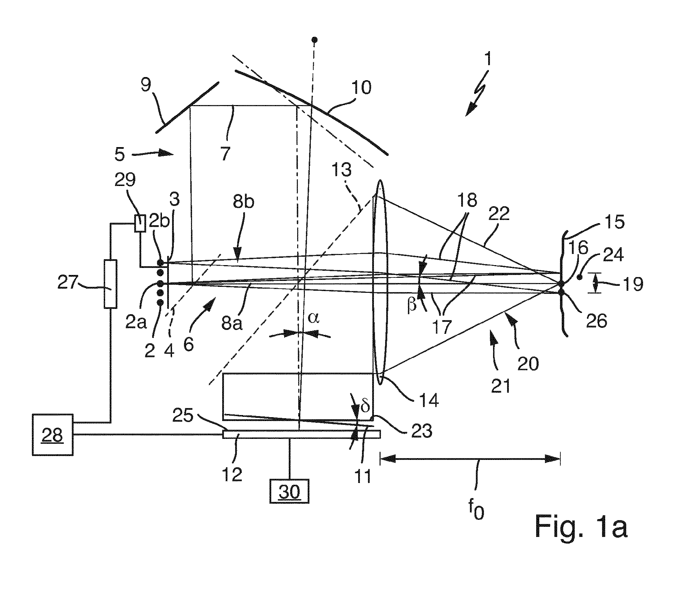

Figur 1a zeigt eine erste Ausführungsform einer Vorrichtung 1 gemäß eines Mach-Zehnder Aufbaus mit mehreren Laser-Emittern 2, u.a. mit einem ersten Laser-Emitter 2a und einem zweiten Laser-Emitter 2b. Die Laser-Emitter 2 sind auf einem Emitter-Chip 3 angeordnet. Sie weisen bevorzugt einen Abstand von kleiner gleich 1 mm bis kleiner 0,1 mm zueinander auf. Sie sind als Multilaserdioden ausgebildet.

In Figur 1a sind beispielhaft fünf Laser-Emitter 2 zu erkennen, die auf dem Emitter-Chip 3 angeordnet sind. Es ist aber auch denkbar, bis zu zweiunddreißig oder mehr Laser-Emitter 2 auf einem Emitter-Chip 3 anzuordnen.

Jeder Laser-Emitter 2 sendet Laserstrahlung mit einer einzigen Wellenlänge aus. Die Wellenlängen der einzelnen Laser-Emitter 2 unterscheiden sich, so dass jeder Laser-Emitter 2 Laserstrahlung mit einer unterschiedlichen Wellenlänge erzeugt. Bevorzugt unterscheiden sich die Wellenlängen der Laserstrahlung zweier benachbarter Laser-Emitter 2 nur gering, bspw. um 1 nm. Bei der Anordnung von 8 oder 16 Laser-Emittern 2 auf einem Emitter-Chip 3 bedeutet das eine spektrale Breite von 8 bzw. 16 nm.

Die Vorrichtung 1 umfasst eine als erster Strahlteiler 4 ausgebildete optische Einrichtung, der die Laserstrahlung der Laser-Emitter 2 jeweils in Referenzstrahlung 5 und Beleuchtungsstrahlung 6 aufteilt. Der erste Strahlteiler 4 kann ein teildurchlässiger Spiegel sein, der die Referenzstrahlung 5 reflektiert und die Beleuchtungsstrahlung 6 transmittiert.

In Figur 1a ist die Referenzstrahlung 5 in Form eines Referenzstrahls 7 des auf dem Emitter-Chip 3 angeordneten ersten Laser-Emitters 2a beispielhaft dargestellt. Die Beleuchtungsstrahlung 6 ist ebenfalls für den ersten Laser-Emitter 2a des Emitter-Chips 3 in Form eines Beleuchtungsstrahls 8a und für den zweiten Laser-Emitter 2b in Form eines Beleuchtungsstrahls 8b dargestellt.

Nachdem der Referenzstrahl 7 am ersten Strahlteiler 4 reflektiert wurde, trifft er in seinem weiteren Verlauf auf eine als Spiegel 9 ausgebildete optische Einrichtung und auf eine weitere optische Einrichtung, die als Parabolspiegel 10 ausgeführt ist. Der Spiegel 9 und der Parabolspiegel 10 reflektieren die Referenzstrahlung 5 derart, dass sie zum einen den Spiegel 10 nahezu kollimiert verlässt und zum anderen unter einem von null Grad verschiedenen Referenzeinfallswinkel α als ebenen Welle 11 auf einen Detektor 12 auftrifft. Der Detektor 12 ist bevorzugt als hochauflösender 2D-Flächensensor ausgebildet.

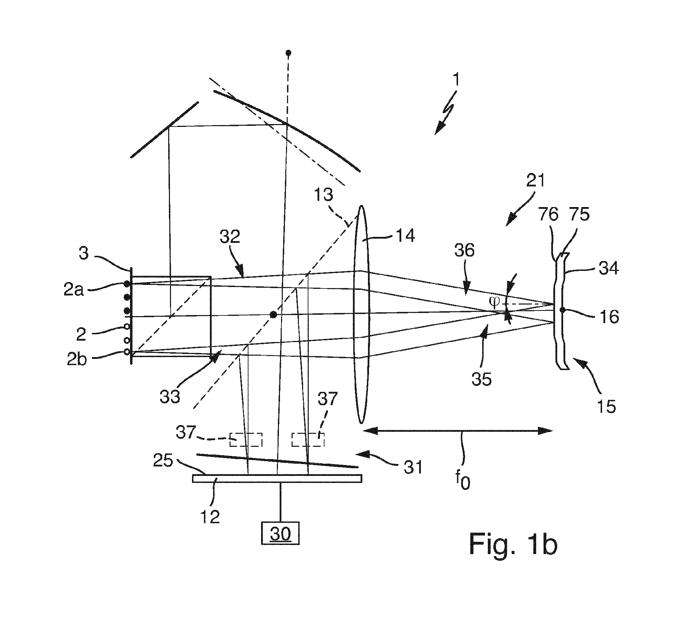

Der Spiegel 9 und der Parabolspiegel 10 sind ausgebildet, die Referenzstrahlung 5 der einzelnen Laser-Emitter 2 derart zu reflektieren, dass die Referenzstrahlung 5 mit unterschiedlichen Wellenlängen, d. h. von unterschiedlichen Laser-Emittern 2 kommend, mit unterschiedlichen Referenzeinfallswinkeln α auf den Detektor 12 treffen. In Figur 1 ist der Parabolspiegel 10 in zwei unterschiedlichen Positionen, einmal als unterbrochene und einmal als durchgezogene Linie, dargestellt. Durch Positionierung des Parabolspiegels 10 können die Referenzeinfallswinkel α der Referenzstrahlung 5 beeinflusst werden.

Zwischen dem Parabolspiegel 10 und dem Detektor 12 ist eine als zweiter Strahlteiler 13 ausgebildete optische Einrichtung angeordnet, die den Referenzstrahl 7 etwas abgeschwächt passieren lässt.

Die Beleuchtungsstrahlen 8a, 8b werden nach dem ersten Strahlteiler 4 ebenfalls durch den zweiten Haupt-Strahlteiler 13 leicht abgeschwächt transmittiert und treffen auf eine als Linse 14 ausgebildete optische Einrichtung. Die Linse 14 leitet die exemplarisch gezeigten Beleuchtungsstrahlen 8a, 8b auf ein Objekt 15 mit einem Objektpunkt 16. Der Objektpunkt 16 liegt idealerweise im Fokus f₀ der Linse 14 oder in einer Fokuszone um den Fokus f₀. Das Objekt 15 kann auch dann noch dreidimensional erfasst werden, wenn der Objektpunkt 16 außerhalb der Fokuszone liegt.

Die Ausdehnung einer Fokuszone der Linse 14 entlang der optischen Achse um den Fokalpunkt der Linse 14 (in Figur 1a der Objektpunkt 16) ist von der Wellenläge der Laserstrahlung, die durch die Linse 14 transmittiert wird, und von der numerischen Apertur der Linse 14 abhängig. Geht man vorliegend von einer numerischen Apertur der Linse 14 von ca. 0,2 aus, liegt die Ausdehnung der Fokuszone, beginnend im Fokalpunkt der Linse 14 entlang der optischen Achse in Richtung des Objekts bei ca. +/- 15 µm.

Der als leicht kugelförmig von einem Laser-Emitter 2 emittierte Beleuchtungsstrahl 8a wird mithilfe der Linse 14 in eine ebene Welle transformiert. In Figur 1 ist dies an Strahlrändern 17 des Beleuchtungsstrahls 8a zu erkennen, die nach der Linse 14 parallel zueinander verlaufen. Der Beleuchtungsstrahl 8a trifft als nahezu ebene Welle und in einem annähernd rechten Winkel, d. h. mit einem Einfallswinkel von ca. 0 Grad, auf das Objekt 15 auf.

Beim Durchgang des Beleuchtungsstrahls 8b durch die Linse 14 erfolgt im Gegensatz zum Beleuchtungsstrahl 8a nicht nur die Transformation in eine ebene Welle. Der Beleuchtungsstrahl 8b wird zusätzlich durch die Linse 14 so abgelenkt, dass er unter einem Einfallswinkel β, der sich von dem Einfallswinkel des Beleuchtungsstrahls 8a unterscheidet, auf das Objekt 15 trifft. Aufgrund der unterschiedlichen Anordnung der Laser-Emitter 2 relativ zu der Linse 14 fällt die Beleuchtungsstrahlung 5 der einzelnen Laser-Emitter 2 mit unterschiedlichen Einfallswinkel β auf das Objekt 15 ein. Bevorzugt treffen alle Beleuchtungsstrahlen 8a, 8b der einzelnen Laser-Emitter 2 in einem rechteckigen Beleuchtungsstreifen 19 auf das Objekt 15.

Es versteht sich, dass das Objekt 15 eine endliche Anzahl an Objektpunkten 16 besitzt, die alle die Laserstrahlung reflektieren. Im Folgenden soll die Reflexion eines Beleuchtungsstrahls 8a, 8b beispielhaft an ausgewählten Objektpunkten, u.a. an dem Objektpunkt 16, und der weitere Strahlverlauf in Richtung Detektor 12 erläutert werden. Die an dem Objekt 15 reflektierten Beleuchtungsstrahlen 8a, 8b bzw. die an einem Objekt 15 reflektierte Beleuchtungsstrahlung 6, werden im Folgendem als Objektstrahl 20 bzw. Objektstrahlung 21 bezeichnet.

Unabhängig von dem Einfallswinkel β eines Beleuchtungsstrahls 8a, 8b wird dieser als Objektstrahl 20 mit Randstrahlen 22 in Form einer Kugelwelle von dem Objektpunkt 16 zurückgeworfen. Der Objektstrahl 20 trifft auf die Linse 14 und wird von dieser in eine nahezu ebene Welle 23 transformiert. Dies gilt für den Fall, dass der Objektpunkt 16 innerhalb der oben beschriebenen Fokuszone oder, wie in Fig.1 gezeigt, im Brennpunkt f₀ der Linse 14 liegt. Befindet sich ein Objektpunkt 24 außerhalb des der Fokuszone, wird ein vom Objektpunkt 24 ausgesandter Objektstrahl 20 mittels der Linse 14 in eine leicht gekrümmte Welle transformiert.

Der Objektstrahl 20 fällt auf den zweiten Strahlteiler 13, der den Objektstrahl 20 derart reflektiert, dass dieser mit einem Einfallswinkel γ von ca. null Grad, d. h. im 90° Winkel zu einer Detektoroberfläche 25 des Detektors 12, auf diese auftrifft. Der Objektstrahl 20 als ebene Welle 23 ist in Figur 1a als horizontale Linie dargestellt, die parallel zu der Detektoroberfläche 25 verläuft.

Der Einfallswinkel γ des Objektstrahls 20 hängt von der Position des Objektpunkts 16, 24 ab. Liegt dieser, wie der Objektpunkt 16 in Figur 1, auf der optischen Achse der Linse 14, dann trifft der Objektstrahl 20, wie bereits beschrieben, mit einem Einfallswinkel γ von 0° auf die Detektoroberfläche 25 auf. Eine Reflexion an einem anderen, nicht auf der optischen Achse angeordnetem Objektpunkt, bspw. an dem Objektpunkt 26, führt zu einem von Null verschiedenen Einfallswinkel γ beim Einfall auf die Detektoroberfläche 25.

Während der Objektstrahl 20 unter dem Einfallswinkel γ auf die Detektoroberfläche 25 trifft, fällt der Referenzstrahls 7 unter dem Referenzeinfallswinkel α auf der Detektoroberfläche 25 ein. Die Referenzeinfallswinkel α und der Einfallswinkel γ unterschieden sich um einen von Null verschiedenen Differenzwinkel δ.

Da der Referenzstrahl 7 und der Objektstrahl 20 derselben Wellenlänge nach dem zweiten Strahlteiler 13 auf ihrem Weg in Richtung der Detektoroberfläche 25 aufeinander treffen, interferieren sie miteinander. Durch diese Interferenz entsteht in Abhängigkeit des Differenzwinkels δ ein Interferenzmuster einer bestimmten räumlichen Frequenz, das mithilfe des Detektors 12 aufgenommen wird. Eine räumliche Frequenz ergibt sich durch die (quasi-) sinusförmige Oszillation der Signalamplituden, welche durch konstruktive (Signalanhebung) und destruktive (Signalabschwächung) Interferenz der Referenzstrahlung 5 und Objektstrahlung 21 einer Wellenlänge entstehen.

Pro Objektpunkt 16, 24, 26 wird bei unterschiedlichen Wellenlängen ein jeweils anderer Differenzwinkel δ gebildet, da sich der Referenzeinfallswinkel α in Abhängigkeit von den Positionen der Laser-Emitter 2 ändert. Die Beleuchtungsfläche auf dem Objekt 15, die Anzahl der Laser-Emitter 2, deren Abstände (und damit die Referenzeinfallswinkel α) und die Pixel-Beschaffenheit des 2D Flächendetektors 12 lassen sich so aufeinander abstimmen, dass keine Doppeldeutigkeiten bei den räumlichen Frequenzen entstehen.

Voraussetzung bei einer Aufnahme der Interferenzmuster durch den Detektor 12 ist, dass die Laserstrahlung, deren Interferenzmuster aufgenommen werden soll, mit im Wesentlichen konstanter Wellenlänge von den jeweiligen Laser-Emittern 2 ausgesendet wird. Hierzu weist die Vorrichtung 1 eine Messeinrichtung auf, die bevorzugt als Fabry Perot Interferometer 27 ausgebildet ist. Das Fabry Perot Interferometer 27 ist aus dem Stand der Technik bekannt und ermöglicht, die Wellenlängen der von den Laser-Emittern 2 ausgesandten Laserstrahlung kontinuierlich zu messen. Das Fabry Perot Interferometer 27 ist mit einer Steuereinrichtung 28 verbunden, die im Falle konstanter Wellenlängen den Detektor 12 ansteuert und eine Aufnahme der Laserstrahlung durch den Detektor 12 auslöst.

Optional kann die Vorrichtung 1 eine Regeleinrichtung 29 aufweisen, die in Abhängigkeit der Messergebnisse des Fabry Perot Interferometers 27 die Laser-Emitter 2 derart regelt, dass die Wellenlängen der emittierten Laserstrahlung im Wesentlichen konstant ist.

Die Interferenzmuster werden für jeden Laser-Emitter 2 und damit für jede Wellenlänge durch den Detektor 12 aufgenommen und mithilfe einer Verarbeitungseinrichtung 30 analysiert und ausgewertet. Die Verarbeitungseinrichtung 30 transformiert das Interferenzmuster, bestehend aus verschiedenen Raumfrequenzen, in den Frequenzbereich, welcher die 3D Bildinformation enthält.

Im Folgenden soll die Auswertung eines Interferenzmusters näher erläutert werden, das durch Reflexion der Beleuchtungsstrahlung 6 eines Laser-Emitters 2 an einem Objektpunkt 16, 24, 26 und durch Interferenz mit der zugehörigen Referenzstrahlung 5 entstanden ist.

Befindet sich der Objektpunkt 16, 26 im Fokus f₀ bzw. im Fokusbereich der Linse 14, d.h. ist der Objektstrahl 21 nach Durchgang durch die Linse 14 als quasi-ebene Welle 23 ausgebildet, wird für die Transformation in den Frequenzbereich bevorzugt die Fourier-Transformation verwendet. Liegt der Objektpunkt 24 außerhalb der Fokuszone, d.h. ist der Objektstrahl 20 nach Passieren der Linse 14 als leicht gekrümmte Welle ausgebildet, wird eine Transformation in den Frequenzbereich bevorzugt mithilfe der Fresnel-Transformation durchgeführt.

Im Frequenzbereich erfolgt die Auswertung der Interferenzmuster hinsichtlich zweier Gesichtspunkte. Zum einen wird die Position des Objektpunkts 16, 24, 26 auf dem Objekt 15 unter Zuhilfenahme der Referenzstrahlung 5 und damit in Abhängigkeit von der Position des Laser-Emitters 2, dessen Laserstrahlung zu dem vorliegenden Interferenzmuster geführt hat, bestimmt. Zum anderen wird für jeden Objektpunkt 16, 24, 26 eine Tiefeninformation ermittelt. Unter dem Begriff Tiefeninformation wird im Rahmen der vorliegenden Erfindung die Position des Objektpunkts 16, 24, 26 in der Tiefe, d.h. in Figur 1a entlang der optischen Achse der Linse 14, verstanden. Sie dient dazu, um Erhebungen oder Vertiefungen auf dem Objekt 15 zu erkennen.

Die Bestimmung der Position des Objektpunkts 16, 24, 26 und der Position des jeweiligen Laser-Emitters 2 erfolgt mithilfe des Differenzwinkels δ. Da der Einfallswinkel γ des Objektstrahls 20 und damit auch der Differenzwinkel δ von der Position des Objektpunkts 16, 24, 26 abhängt, entstehen in Abhängigkeit der Position des Objektpunktes 16, 24, 26 unterschiedliche für die Position des Objektpunktes 16, 24, 26 charakteristische Interferenzmuster bestimmter räumlicher Frequenzen. Dabei ist die Breite des Frequenzbandes, in dem sich die Änderungen der räumlichen Frequenz in Abhängigkeit der Position des Objektpunkts 16, 24, 26 bewegt, für jeden Laser-Emitter 2 eng eingegrenzt.

Der Differenzwinkel δ ist darüber hinaus von der Position des jeweiligen Laser-Emitters 2 auf dem Emitter-Chip 3 abhängig. Wie bereits weiter oben beschrieben, trifft die Referenzstrahlung 5 der jeweiligen Laser-Emitter 2 in Abhängigkeit von der Position des jeweiligen Laser-Emitters 2 mit unterschiedlichen Referenzeinfallswinkeln α auf den Detektor 12 auf. Somit bewirkt jeder Laser-Emitter 2 eine deutlich andere räumliche Frequenz als bspw. der benachbarte Laser-Emitter 2. Durch diese deutlichen Unterschiede der räumlichen Frequenzen, basierend auf der Position unterschiedlicher Laser-Emitter 2, werden die Unterschiede nicht mit den Änderungen, basierend auf unterschiedlichen Objektpunkten 16, 24, 26, verwechselt. Jede Laser-Emitter Position erlaubt ein Band von Raumfrequenzen, welches wiederum gerade die Anzahl der Objektpunkte pro Emitter 2 abdecken kann. Für einen benachbarten Emitter 2 ergibt sich daran anschließend wieder ein Band von Raumfrequenzen, usw. Damit kann - nach Transformation (z.B. Fourier, Fresnel) - einem bestimmten lateralen Objektpunkt 16, 24, 26 über die Phasenwerte der lokalen Interferenzmuster eine Tiefeninformation zugewiesen werden.

Kombiniert man nun die Information der Position des Objektpunkts 16, 24, 26, die Information über den jeweiligen Laser-Emitter 2 und die Tiefeninformation des jeweiligen Objektpunkts 16, 24, 26 miteinander, kann eine 3D-Struktur des Objekts 15 ermittelt werden.

Bei einer realen Messung senden alle Laser-Emitter 2 gleichzeitig Laserstrahlung aus, wodurch mehrere Interferenzmuster auf der Detektoroberfläche 25 entstehen Alle Interferenzmuster werden zeitgleich vom Detektor 12 aufgenommen und von der Verarbeitungseinrichtung 30 ausgewertet. Die 3D-Struktur des Objekts 15 wird dann basierend auf diesen Auswertungen aller Interferenzmuster generiert.

Die Vorrichtung 1 kann bevorzugt zusätzlich Schichtdicken von Schichten (nicht dargestellt) messen, die auf dem Objekt 15 angeordnet sind. Befindet sich eine Schicht auf dem Objekt 15, werden aufgrund der unterschiedlichen Wellenlängen der von den Laser-Emittern 2 ausgesandten Laserstrahlung die Beleuchtungsstrahlen 8a, 8b zum Teil an der Oberfläche der Schicht reflektiert, während der andere Teil der Beleuchtungsstrahlen 8a, 8b die Schicht durchdringen und an der Oberfläche des Objekts 15 reflektiert werden. Bei der Auswertung der Interferenzmuster im Frequenzbereich liegen für jeden Objektpunkt 16, 24, 26 zwei leicht unterschiedliche Tiefeninformationen vor. Anhand der unterschiedlichen Tiefeninformationen kann auf die Schichtdicke geschlossen werden. Die Vorrichtung 1 umfasst somit eine inhärente Messeinheit zur Bestimmung von Schichtdicken, wobei die Messeinheit als Teil der Verarbeitungseinrichtung 30 ausgebildet sein kann. Die geringste mit der Vorrichtung 1 messbare Schichtdicke ist durch die spektrale Breite der eingestrahlten Wellenlängen in der Wellenlängen-Gruppe gegeben.

Figur 1b zeigt eine zweite Ausführungsform der Vorrichtung 1. Die Vorrichtung 1 weist eine Messeinheit 31 auf, mit der die an einer zu vermessenden Schicht 75 reflektierte Objektstrahlung 21 der Laser-Emitter 2 gemessen werden kann. Im Gegensatz zur Vorrichtung 1 aus Figur 1a arbeitet die Messeinheit 31 basierend auf dem Prinzip der Ellipsometrie und kann so die Dicke der auf dem Objekt angeordneten Schicht 75 ermitteln. Das Verfahren der Ellipsometrie zur Bestimmung von Schichtdicken ist aus dem Stand der Technik bekannt.

Die Messeinheit 31 umfasst die Laser-Emitter 2. Im Gegensatz zur Figur 1a sind die Laser-Emitter in Figur 1b unterschiedlich polarisiert. Beispielsweise sind die in Figur 1b gezeigten drei oberen Laser-Emitter 2, 2a auf dem Emitter-Chip 3 in einer ersten Art polarisiert, z.B. P-polarisiert. Die in Figur 1b unteren drei Laser-Emitter 2, 2b weisen eine zweite Polarisation, z.B. eine S-Polarisierung, auf, die von der ersten Polarisation verschieden ist.

Beispielhaft sind ein erster Beleuchtungsstrahl 32 des in Figur 1b obersten Laser-Emitters 2a und ein zweiter Beleuchtungsstrahl 33 des in Figur 1b untersten Laser-Emitters 2b auf dem Emitter-Chip 3 dargestellt. Im Folgenden sollen die Strahlverläufe der beiden Beleuchtungsstrahlen 32, 33 näher erläutert werden.

Der erste Beleuchtungsstrahl 32 wird durch die Linse 14 in eine ebene Welle transformiert und trifft unter einem Einfallswinkel ϕ auf die Oberfläche 76 der Schicht 75 des Objekts 15 auf. Teile des Beleuchtungsstrahls 32 werden als Objektstrahlung 21 an der Oberfläche 76 der Schicht 75 reflektiert. Teile der Beleuchtungsstrahlung 33 durchdringen die Schicht 75 und werden von einer Oberfläche 34 des Objekts 15, auf dem sich die Schicht 75 befindet, ebenfalls als Objektstrahlung 21 zurückgeworfen. Der als Objektstrahlung 21 reflektierte Beleuchtungsstrahl 33 ist in Figur 1b als erster Objektstrahl 35 dargestellt.

Der erste Objektstrahl 35 wird als ebene Welle an der Oberfläche 34 und der Oberfläche 76 reflektiert und trifft nach der Reflexion abermals auf die Linse 14. Diese bündelt den ersten Objektstrahl 35, der im weiteren Verlauf an dem zweiten Strahlteiler 13 reflektiert wird und als Punktstrahl auf die Detektoroberfläche 25 des Detektors 12 trifft. Der Detektor 12 detektiert den als Punktstrahl auf ihn treffenden Objektstrahl 35.

Bei dem beschriebenen Strahlverlauf wird von einer sehr glatten Oberfläche 76 der Schicht 75 und einer sehr glatten Oberfläche 34 des Objekts 15 ausgegangen, an denen der erste Beleuchtungsstrahl 32 reflektiert wird. Bei leicht gekrümmter Oberfläche 76 bzw. 34 würde sich der auf den Detektor 12 gerichtete Punktstrahl entsprechend verbreitern.

Der zweite Beleuchtungsstrahl 33 verläuft spiegelbildlich zu dem ersten Beleuchtungsstrahl 32. Dadurch sind der zweite Beleuchtungsstrahl 33 und der erste Objektstrahl 35 zumindest teilweise überlagert. Der zweite Beleuchtungsstrahl 33 wird als zweiter Objektstrahl 36 an den Oberflächen 76, 34 reflektiert, durch die Linse 14 gebündelt und durch den zweiten Strahlteiler 13 derart reflektiert, dass er als Punktstrahl auf die Detektoroberfläche 25 des Detektors 12 auftrifft. Der Detektor 12 detektiert den als Punktstrahl auf ihn treffenden Objektstrahl 36.

Basierend auf den Objektstrahlen 35, 36 kann dann die Dicke einer auf dem Objekt 15 befindlichen Schicht 75 auf bekannte Art und Weise mithilfe der Verarbeitungseinrichtung 30 ermittelt werden.

Grundsätzlich ist es auch vorstellbar, dass die Schicht 75 und das Objekt 15 raue Oberflächen 76, 34 aufweisen, die, wie in Figur 1a dargestellt, die Objektstrahlung 21 als Kugelwelle reflektieren. Ein solches Verfahren ist ebenfalls aus dem Stand der Technik bekannt. In diesem Fall wird jeder Oberflächenpunkt (nicht dargestellt) auf der Oberfläche 76 der Schicht 75 und jeder Objektpunkt 16 des Objekts 15 als Streupunkt aufgefasst (vgl. Figur 1a). Dadurch werden sämtliche Informationen eines jeden Oberflächenpunktes und Objektpunktes 16, im Gegensatz zur glatten Oberfläche, auf die gesamte Detektoroberfläche 25 abgebildet.

Optional kann die Vorrichtung 1 einen oder mehrere Analysatoren 37 umfassen, die zwischen dem zweiten Strahlteiler 13 und der Detektoroberfläche 25 angeordnet sind. Die Analysatoren 37 sind als Polarisatoren ausgebildet, mit denen der Polarisationszustand von Laserstrahlung ermittelt werden kann.

Werden Analysatoren 37 verwendet, senden die Laser-Emitter 2 Laserstrahlung mit einem Polarisationswinkel von bevorzugt 45 Grad aus, so dass die Beleuchtungsstrahlen 32, 33 linear polarisiert auf die Schicht 75 bzw. das Objekt 15 einstrahlen. Nach Reflexion derselben an der Schicht 75 bzw. dem Objekt 15 treffen die Objektstrahlen 35, 36 auf die zu den Positionen der Emitter 2a und 2b konjugiert angeordneten Analysatoren 37, die die Polarisation der Objektstrahlen 35, 36 ermitteln. Beispielsweise werden die Laserstrahlung des Laser-Emitters 2a und die Laserstrahlung des Laser Emitters 2b durch die Schichtdicke der Schicht 75 unterschiedlich polarisiert. Die Objektstrahlen 35, 36 fallen anschließend auf den Detektor 12, der diese detektiert. In diesem Zusammenhang bedeutet konjungiert zu den Positionen der Emitter 2a und 2b angeordnet, dass die Analysatoren 37 entsprechend dem Strahlenverlauf der von den Laser-Emittern 2a, 2b emittierten Laserstrahlung angeordnet sind.

Es ist weiter denkbar, die Linse 14 als zwei Teillinsen auszuführen, wobei jeweils einer der beiden Beleuchtungsstrahlen 32, 33 durch jeweils eine der beiden Teillinsen transmittiert wird. Mithilfe zusätzlicher Spiegel (nicht dargestellt) werden die Beleuchtungsstrahlen 32, 33 nach den beiden Teillinsen auf die Schicht 75 bzw. das Objekt 15 gerichtet. Somit ist es möglich, Einfallswinkel ϕ größer oder gleich 30 Grad, bevorzugt nahe 57 Grad zu realisieren. Dadurch können auch Schichtdicken unter 100 nm gemessen werden.

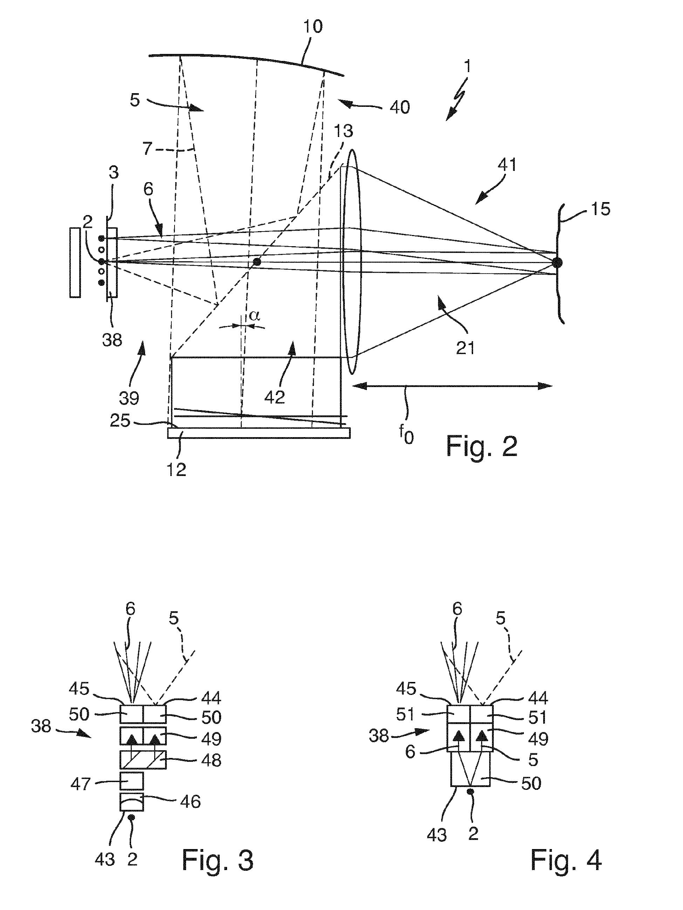

Figur 2 zeigt die Vorrichtung 1 gemäß einer dritten Ausführungsform in einem Michelson-Aufbau. Diese Vorrichtung 1 unterscheidet sich von der in Figur 1a gezeigten Vorrichtung 1 durch eine als mikro-optisches Array 38 ausgebildete optische Einrichtung. Das mikro-optische Array 38 umfasst die Funktion des in Figur 1a dargestellten ersten Strahlteilers 4. Darüber ist es mithilfe des mikro-optischen Arrays 38 möglich, die Referenzstrahlung 5 und die Beleuchtungsstrahlung 6 unterschiedlich zu polarisieren. Das mikro-optische Array 38 teilt die Laserstrahlung der Laser-Emitter 2 in die Referenzstrahlung 5 und die Beleuchtungsstrahlung 6 auf, wobei die Referenzstrahlung 5 gegenüber der Beleuchtungsstrahlung 6 eine deutlich größere numerische Apertur aufweist.

Darüber hinaus wurde bei der Vorrichtung 1 gemäß Figur 2 im Vergleich zu der Vorrichtung 1 aus Figur 1 a auf den Spiegel 9 verzichtet. Der Referenzstrahl 7 eines Laser-Emitters 2 wird am Strahlteiler 13, der als Strahlteiler-Spiegel ausgebildet ist, reflektiert und auf den Parabolspiegel 10 geleitet. Dieser reflektiert den Referenzstrahl 7 derart, dass er mit einem Referenzeinfallswinkel α auf die Detektoroberfläche 25 des Detektors 12 trifft.

Es ist auch denkbar den Strahlteiler-Spiegel 13 als polarisierenden Strahlteiler auszubilden, der in Abhängigkeit der Polarisationszustände der Laserstrahlung diese reflektiert oder transmittiert. Beispielsweise wird die Referenzstrahlung 5 reflektiert und die Beleuchtungsstrahlung 6 aufgrund ihres anderen Polarisationszustands transmittiert.

Die Reflexionseigenschaft des Parabolspiegels 10 in Bezug auf den Einstrahlwinkel α kann entweder erreicht werden, indem, wie in Figur 2 gezeigt, der Parabolspiegel 10 relativ zur Detektoroberfläche 25 leicht geneigt ist. Es ist aber auch denkbar, den Parabolspiegel 10 relativ zur Detektoroberfläche 25 nicht zu neigen und den Emitter-Chip 3 mit den Laser-Emittern 2 so lateral anzuordnen, dass die Referenzstrahlung 5 immer unter einem von Null verschiedenen Referenzeinfallswinkel α auf den Detektor 12 trifft. Ein laterales Anordnen der Laser-Emitter 2 bedeutet in diesem Zusammenhang ein Verschieben derselben in eine in Figur 2 vertikale Richtung und/oder ein Verschieben quer zur der Bildebene in Figur 2.

Der Parabolspiegel 10 kann optional eine zirkular polarisierende Schicht erhalten (nicht gezeigt), welche den Polarisationszustand nach 2-maligem Durchlaufen wieder Richtung Strahlteiler 13 um 90° dreht. Das hat den Vorteil, dass möglichst viel Licht den Detektor 12 erreicht.

Bis auf die eben beschriebenen Unterschiede verläuft die Laserstrahlung eines Laser-Emitters 2 wie bereits anhand der Vorrichtung 1 in Figur 1a beschrieben. Auch die Auswertung der Interferenzmuster auf dem Detektor 2 erfolgt analog zu der Vorrichtung 1 aus Figur 1a.

Der Strahlteiler 13 definiert in Figur 2 vier optische Arme, die im Folgenden zum besseren Verständnis des Michelson-Aufbaus erläutert werden sollen. Der erste optische Arm ist definiert als Beleuchtungsarm 39, in dem sich der Emitter-Chip 3 mit den Laser-Emittern 2 und das mikro-optische Array 38 befindet. Im Beleuchtungsarm 39 verläuft die Beleuchtungsstrahlung 6 in Richtung des Strahlteilers 13.

Als zweiten optischen Arm definiert der Strahlteiler 13 einen Referenzarm 40, in dem die Referenzstrahlung 5, von dem Strahlteiler 13 reflektiert, in Richtung des Parabolspiegels 10 und von diesem wieder zurück in Richtung des Strahlteilers 13 gelenkt wird. In dem Referenzarm 40 befindet sich vorzugsweise der Parabolspiegel 10.

Der dritte optische Arm wird Objektarm 41 genannt. In ihm ist die Linse 14 und das Objekt 15 angeordnet. Gegebenenfalls können sich noch weitere Linsen oder optische Elemente im Objektarm 41 befinden. Im Objektarm 41 wird die Beleuchtungsstrahlung 6 auf das Objekt 15 gelenkt und die vom Objekt 15 reflektierte Objektstrahlung in Richtung des Strahlteilers 13 geführt.

Der vierte optische Arm ist ein Detektionsarm 42. Er ist definiert als die Wegstrecke vom Strahlteiler 13 bis zum Detektor 12. Der Detektionsarm 42 schließt den Detektor 12 und gegebenenfalls weitere Strahlteiler ein, z.B. falls mehrere Detektoren 12 zum Einsatz kommen.

Bevorzugt können optische polarisationsdrehende Elemente in dem Objektarm 41 und Referenzarm 40 zum Einsatz kommen. In jedem Falle ist bei polarisierten Beleuchtungs- und Referenzstrahlen sicher zu stellen, dass vor dem Detektor 12 eine Analysator (Polarisator, nicht dargestellt) die Lichtstrahlung wieder auf eine gemeinsame interferenzfähige Ebene bringt. Werden keine polarisierenden Elemente (einschl. im mikro-optischen-Array 38) verwendet, ist mit erhöhtem "Crosstalk" (Störeinflüssen) zwischen Objektstrahlung 21 und Referenzstrahlung 5 zu rechnen, der aufwendiger korrigiert werden muss.

Figur 3 zeigt eine Detailansicht des mikro-optischen Arrays 38 aus Figur 2 in einer ersten Ausführungsform von der Seite. Das mikro-optische Array 38 kann prinzipiell nicht nur im Michelson Aufbau gemäß Figur 2 sondern auch optional im Mach-Zehnder Aufbau gemäß Figur 1a zwischen den Laser-Emittern 2 und weiteren optischen Einrichtungen der Vorrichtung 1 (Figur 1 a) positioniert werden.

Die Laserstrahlung jedes einzelnen Laser-Emitters 2 durchläuft das mikro-optische Array 38. Beispielhaft wird im Folgenden der Aufbau des mikro-optischen Arrays 38 für einen Laser-Emitter 2 erläutert. Der Aufbau des mikro-optischen Arrays 38 für die weiteren Laser-Emitter 2 ist identisch.

Das mikro-optische Array 38 weist einen Eingang 43, einen ersten Ausgang 44 und einen zweiten Ausgang 45 auf, über die die Laserstrahlung das mikro-optische Array 38 wieder verlässt.

Nachdem die Laserstrahlung von den Laser-Emittern 2 über den Eingang 43 in das mikro-optische Array 38 eingestrahlt wurde, durchläuft sie eine stabförmige Zylinderlinse 46, die die Laserstrahlung derart bündelt, so dass eine nahezu zirkulare Laserstrahlung mit einer numerischen Apertur von bevorzugt ca. 0,1 entsteht.

In Strahlrichtung hinter der Zylinderlinse 46 ist ein Polarisator 47 angeordnet, der die Laserstrahlung in Figur 3 senkrecht zur Blattebene polarisiert. Ein Strahlteiler 48 teilt die Laserstrahlung anschließend in die Referenzstrahlung 5 und die Beleuchtungsstrahlung 6 auf. Die so entstandene Referenzstrahlung 5 durchläuft anschließend einen Zirkulator 49, der die Polarisationsebene um 90° dreht, d.h. die Referenzstrahlung 5 parallel zur Papierebene in Figur 3 polarisiert. Die Beleuchtungsstrahlung 6 hingegen wird nicht in ihrer Polarisation beeinflusst. Somit sind die Referenzstrahlung 5 und die Beleuchtungsstrahlung 6 in unterschiedlichen Richtungen polarisiert. Selbstverständlich könnten die Polarisationszustände auch umgekehrt sein, d.h. die Laserstrahlung in Figur 3 wird mittels des Polarisators 47 parallel zur Blattebene polarisiert während ein Polarisieren der Referenzstrahlung 5 durch den Zirkulator 49 senkrecht zur Papierebene erfolgt.

Sowohl die Referenzstrahlung 5 wie auch die Beleuchtungsstrahlung 6 durchlaufen anschließend jeweils ein Hologramm 50, das die Referenzstrahlung 5 mit einer hohen numerischen Apertur und die Beleuchtungsstrahlung 6 im Verhältnis dazu mit einer geringen numerischen Apertur versieht. Damit kann auch die Form des oder der Beleuchtungsstreifen vorgegeben werden.

Nach Durchlaufen der Hologramme 50 treten die Referenzstrahlung 5 und die Beleuchtungsstrahlung 6 durch die Ausgänge 44 bzw. 45 aus dem mikro-optischen Array 38 aus. Es ist allerdings auch prinzipiell möglich die Reihenfolge einzelner optischer Elemente zu vertauschen. Ebenso ist es möglich auch mehrere Hologrammebenen vorzusehen.

Figur 4 zeigt ein mikro-optisches Array 38 gemäß einer zweiten Ausführungsform. Das mikro-optische Array 38 stellt eine Vereinfachung gegenüber dem mikro-optischen Array 38 aus Figur 3 dar. Es unterscheidet sich dadurch, dass der Strahlteiler 48 (Figur 3) als Hologramm 50 ausgebildet ist und das mikro-optische Array 38 darüber hinaus lediglich, einen Zirkulator 49 und einen Doppelpolarisator 51 umfasst.

Laserstrahlung des Laser-Emitters 2 tritt über den Eingang 43 in das mikro-optische Array 38 ein. Im Hologramm 50 wird die Laserstrahlung zunächst in Referenzstrahlung 5 und Beleuchtungsstrahlung 6 aufgespalten. Die Referenzstrahlung 5 wird im Hologramm 50 mit einer hohen numerischen Apertur und die Beleuchtungsstrahlung 6 mit einer im Vergleich dazu geringen numerischen Apertur versehen. Analog kann auch die Form des oder der Beleuchtungsstreifen vorgegeben werden.

In dem nachfolgenden Zirkulator 49 erfolgt eine Polarisierung der Referenzstrahlung 5, die in ihrer Polarisation um 90 Grad gedreht wird. Die Beleuchtungsstrahlung 6 hingegen wird in ihrer Polarisation nicht beeinflusst. Selbstverständlich kann dies auch umgekehrt stattfinden. Im nachfolgenden Doppelpolarisator 51 werden sowohl die Referenzstrahlung 5 wie auch die Beleuchtungsstrahlung 6 polarisiert. Die Polarisationsrichtung der Beleuchtungsstrahlung 6 unterscheidet sich nach wie vor um 90 Grad von der Polarisation der Referenzstrahlung 5. Gemeinsam werden im Doppelpolarisator 51 sowohl in der Referenzstrahlung 5 wie auch in der Beleuchtungsstrahlung 6 nicht gewollt gedrehte Polarisations-Anteile unterdrückt.

Die Referenzstrahlung 5 und die Beleuchtungsstrahlung 6 treten dann an dem jeweiligen Ausgang 44, 45 aus dem mikro-optischen Array 38 aus. Auch hier kann im Prinzip die Reihenfolge der optischen Elemente vertauscht werden, bzw. das Hologramm 50 in zwei einzelne Hologramme mit weniger Einzelaufgaben aufgespalten werden.

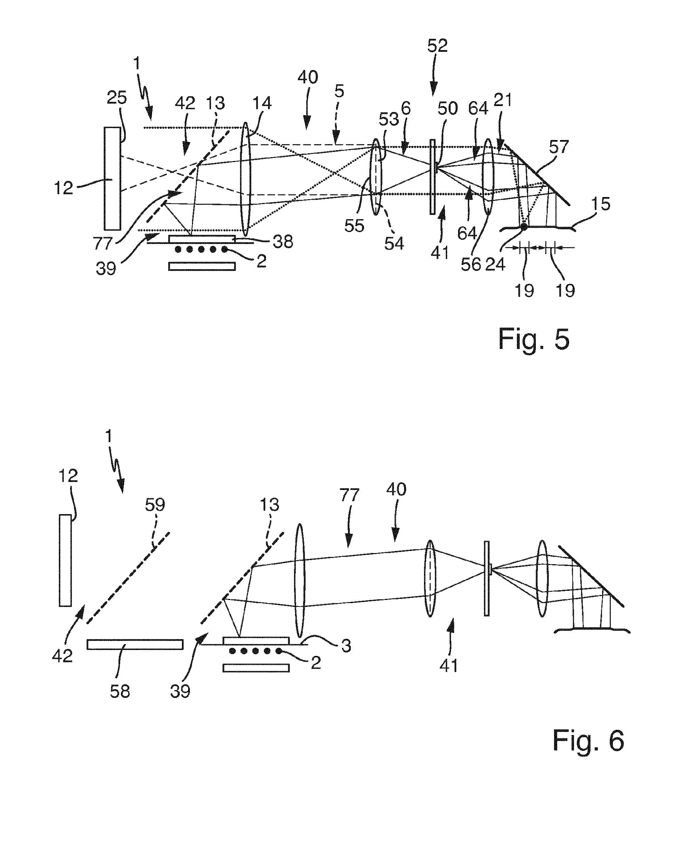

In Figur 5 ist die erfindungsgemäße Vorrichtung 1 gemäß einer vierten Ausführungsform dargestellt. Ihr Aufbau entspricht dem Michelson-Aufbau aus Figur 2, wobei die Positionen der Laser-Emitter 2 und des Detektors 12 relativ zu dem Strahlteiler 13 vertauscht wurden. Darüber hinaus ist der Strahlteiler 13 als polarisierender Strahlteiler ausgeführt, d.h. er transmittiert oder reflektiert Strahlung in Abhängigkeit der Polarisation.

Zusätzlich zu den bisher beschriebenen Vorrichtungen 1 weist die Vorrichtung 1 gemäß Figur 5 eine Optik 52 auf, die mehrere optische Einrichtungen umfasst. Die Optik 52 erstreckt sich von der Linse 14 bis zum objektseitigen Ende der Vorrichtung 1. Eine der optischen Einrichtungen ist als zweite Linse 53 mit einem Rückreflektor 54 ausgebildet. Auf den Rückreflektor 54 treffende Laserstrahlung wird bevorzugt zu 90 % transmittiert und zu 10 % reflektiert. Der Rückreflektor 54 zusammen mit der Vorderseiten-Krümmung der Linse 53 übernimmt somit die Funktion des Parabolspiegels 10 aus Figur 2.

An einer Vorderseite 55 der zweiten Linse 53 befindet sich ein Zirkular-Polarisator (nicht dargestellt), mit dessen Hilfe auf die Linse 53 treffende Referenzstrahlung 5, und die Objektstrahlung 21 in ihrer Polarisation um 90° gedreht wird. Dadurch wird erreicht, dass die komplette rückgestrahlte Laserstrahlung nach Transmission der Linse 14 an dem polarisierenden Strahlteiler 13 transmittiert wird.

Eine weitere optische Einrichtung der Optik 52 ist als Hologramm 50 ausgebildet, das die Beleuchtungsstrahlung 6 der Laser-Emitter 2 derart ablenkt, dass sie als Beleuchtungsstreifen 19 auf das Objekt 15 treffen. Insbesondere die numerische Apertur der Beleuchtungsstrahlung 6 eines jeden Laser-Emitters 2 wird mithilfe des Hologramms 50 so verändert, dass zum einen zwei voneinander versetzte Beleuchtungsstreifen 19 entstehen und zum anderen jeder dieser Beleuchtungsstreifen 19 eine annähernd rechteckige Form besitzt. Mittels einer dritten Linse 56, die ebenfalls eine optische Einrichtung der Optik 52 ist, wird die durch das Hologramm 50 abgelenkte Beleuchtungsstrahlung 6 auf einen Spiegel 57 gerichtet, der die Beleuchtungsstrahlung 6 auf das Objekt 15 lenkt.

Aufgrund der im Vergleich zu Figur 2 unterschiedlichen Position der Laser-Emitter 2 und des Detektors 12, wurden auch die Positionen des Beleuchtungsarms 39 und des Detektionsarms 42 im Vergleich zu Figur 2 vertauscht. Im Gegensatz zur Vorrichtung 1 aus Figur 2 überlagern sich bei der Vorrichtung 1 gemäß Figur 5 der Referenzarm 40 und der Objektarm 41 zumindest teilweise.

Im Folgenden soll der Verlauf der Beleuchtungsstrahlung 6 (als durchgehende Linie dargestellt) und der Referenzstrahlung 5 (gestrichelt dargestellt) exemplarisch näher erläutert werden.

Von den Laser-Emittern 2 emittierte Laserstrahlung 77 durchläuft das mikro-optische Array 38 und tritt aus diesem in einem Polarisationszustand, d.h. polarisiert wieder aus. Im mikro-optische Array 38 erfolgt kein Aufspalten der Laserstrahlung 77 in Referenzstrahlung 5 und Beleuchtungsstrahlung 6 (vgl. Figur 3).

Die aus dem mikro-optischen Array 38 austretende Laserstrahlung 77 ist derart linear polarisiert, dass der polarisierende Strahlteiler 13 die Laserstrahlung 77 in Richtung des Objekts 15 durch die Linse 14 reflektiert. Die Linse 14 transformiert die Laserstrahlung 77 in eine ebene Welle. Die Laserstrahlung 77 fällt dann auf die zweite Linse 53 und wird in ihrer Polarisationsebene durch den Zirkular-Polarisator auf der Vorderseite 55 der Linse 53 zirkular polarisiert. Die Laserstrahlung 77 fällt weiter auf den Rückreflektor 54, der einen geringen Anteil also z.B.10 % davon zurück in Richtung des zweiten Strahlteilers 13 reflektiert. Dieser reflektierte Anteil wird aufgrund eines erneuten Durchgangs durch den Zirkulator auf der Vorderseite 55 der Linse 53 nochmals zirkular polarisiert, so dass sein Polarisationszustand effektiv um 90° gedreht wird. Das bedeutet, dass der Polarisationszustand der Laserstrahlung 77 durch zweimaliges durchlaufen des Zirkular-Polarisators um 90° gedreht wird.

Der am Rückreflektor 53 reflektierte Anteil bildet die Referenzstrahlung 5 und trifft wiederum auf die zweite Linse 14. Die Referenzstrahlung 5 wird dann von dem zweiten Strahlteiler 13 aufgrund der geänderten Polarisation transmittiert. Nach dem zweiten Strahlteiler 13 trifft die Referenzstrahlung 5 auf den Detektor 12 und wird von diesem aufgenommen.

Der durch den Rückreflektor 54 transmittierte Anteil der Laserstrahlung 77 bildet die Beleuchtungsstrahlung 6. Der transmittierte Anteil beträgt etwa 90 % der Laserstrahlung 77. Der Rückreflektor 54 ist also als Strahlteiler ausgebildet, der die Laserstrahlung 77 in die Referenzstrahlung 5 und die Beleuchtungsstrahlung 6 aufteilt.

Die Beleuchtungsstrahlung 6 wird mithilfe der zweiten Linse 53 auf das Hologramm 50 fokussiert. Dieses teilt die Beleuchtungsstrahlung 6 in zwei Strahlenbündel 64 auf, die über die dritte Linse 56 und den Spiegel 57 als zwei Beleuchtungsstreifen 19 auf das Objekt 15 gelenkt werden.

Der Verlauf der als Objektstrahlung 21 reflektierten Beleuchtungsstrahlung 6 soll im Folgenden anhand des Objektpunkts 24 erläutert werden. Die vom Objektpunkt 24 reflektierte - und durch den Durchgang des vorherigen Zirkulators zirkular polarisierte - Objektstrahlung 21 (gepunktet dargestellt) trifft auf den Spiegel 57. Dieser lenkt die Objektstrahlung 21 auf die dritte Linse 56, die die Objektstrahlung 21 in eine ebene Welle transformiert. Die Objektstrahlung 21 trifft dann auf die zweiten Linse 53 und damit auf den an der Vorderseite 55 angeordneten Zirkulator, welcher die Objektstrahlung 21 entsprechend der Durchlassrichtung des polarisierenden Strahlteilers 13 nun analog zur Referenzstrahlung polarisiert. Die zweite Linse 53 bildet die Objektstrahlung 21 auf die Linse 14 ab, die diese auf den zweiten Strahlteiler 13 wirft. Dieser transmittiert aufgrund der entsprechenden Polarisierung die Objektstrahlung 21, so dass die Objektstrahlung 21 auf den Detektor 12 trifft. Der Detektor 12 nimmt die Objektstrahlung 21 auf.

Auf dem Detektor 12 interferieren die Referenzstrahlung 5 und Objektstrahlung 21 wie zu Figur 1a beschrieben. Die Auswertung der hierdurch entstehenden Interferenzmuster erfolgt analog zu der Vorrichtung 1 aus Figur 1a. Von welchem Beleuchtungsstreifen 19 die vom Objekt 15 reflektierte Objektstrahlung 6 stammt, kann mithilfe des Einfallswinkels γ und des Differenzwinkels δ (Figur 1a) bei der Auswertung der Objektstrahlung 21 auf der Detektoroberfläche 25 des Detektors 12 ermittelt werden.

Die in Figur 6 gezeigte Vorrichtung 1 gemäß einer fünften Ausführungsform unterscheidet sich von der in Figur 5 gezeigten Vorrichtung 1 durch einen zweiten Detektor 58 und einen zusätzlichen Strahlteiler 59, der Laserstrahlung auf den zweiten Detektor 58 lenkt. Die Vorrichtung 1 gemäß Figur 6 weist zwei Emitter-Chips 3 auf, wobei der in Figur 6 vordere Emitter-Chip 3 den hinteren Emitter-Chip verdeckt. Die Laserstrahlung der auf dem in Figur 6 vorderen Emitter-Chip 3 angeordneten Laser-Emittern 2 trifft auf den Detektor 12. Die Laserstrahlung der auf den in Figur 6 hinteren Emitter-Chip angeordneten Laser-Emitter trifft auf den zweiten Detektor 58.

Die Laser-Emitter 2 jedes Emitter-Chips 3 bilden eine Wellenlängengruppe, wobei jeder Wellenlängengruppe eine zentrale Wellenlänge zugeordnet werden kann. Die zentrale Wellenlänge wird ermittelt, indem der Mittelwert über die unterschiedlichen Wellenlängen der von den Laser-Emittern 2 eines Emitter-Chips 3 ausgesendeten Laserstrahlung gebildet wird.

Der zusätzliche Strahlteiler 59 teilt die auf ihn fallende Laserstrahlung der Laser-Emitter 2 in Abhängigkeit der zentralen Wellenlängen auf. Diese Wellenlängenabhängigkeit des zusätzlichen Strahlteilers 59 ist derart gewählt, dass er die von den Laser-Emittern 2 des in Figur 6 vorderen Emitter-Chips 3 ausgesandten Laserstrahlung mit einer ersten zentralen Wellenlänge transmittiert. Die Laserstrahlung der Laser-Emitter des nicht gezeigten hinteren Emitter-Chips wird dagegen mit einer zweiten zentralen Wellenlänge am zusätzlichen Strahlteiler 59 auf den Detektor 58 reflektiert.

Durch die Verwendung zweier Emitter-Chips 3 trifft die Laserstrahlung 77 leicht geneigt zur Blattebene in Figur 6 auf den Strahlteiler 13 und die weiteren optischen Einrichtungen der Vorrichtung 1 auf.

Projiziert auf die Blattebene in Figur 6 stimmt jedoch der Verlauf der Laserstrahlung 77 im Bereich des Beleuchtungsarms 39, des Referenzarms 40 und des Objektarms 41 mit dem in Figur 5 gezeigtem Verlauf überein. Unterschiede im Strahlverlauf ergeben sich lediglich im Detektionsarm 42 durch den zusätzlichen Strahlteiler 59 wie oben beschrieben.

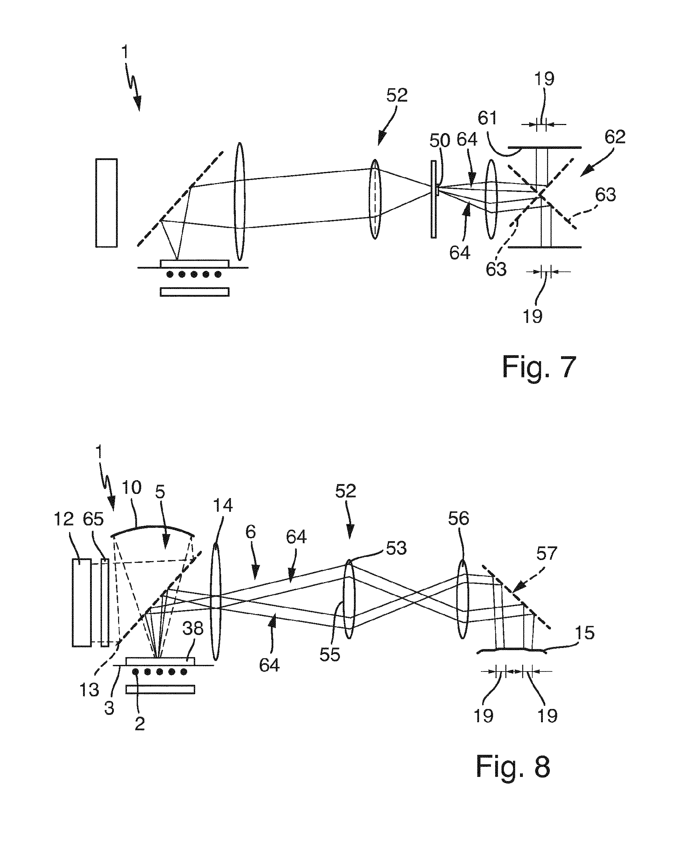

Figur 7 zeigt die erfindungsgemäße Vorrichtung 1 gemäß einer sechsten Ausführungsform, die als Innenbohrungs-Scanner ausgebildet ist. Mit ihm kann ein Innenraum, insbesondere der Innenraum einer Bohrung 62 oder eine Innenwand 61 einer Bohrung 62 untersucht werden. Hierzu weist die Vorrichtung 1 zwei weitere Strahlteiler 63 auf.

Das Hologramm 50 erzeugt analog zu der Vorrichtung 1 aus Figur 5 zwei Strahlenbündel 64, die im Unterschied zu der Vorrichtung 1 aus Figur 5 mithilfe der Strahlteiler 63 in zwei gegenüberliegende Richtungen auf die Innenwand 61 der Bohrung 62 gelenkt werden.

Um die Bohrung 62 über ihren gesamten Umfang hin zu untersuchen, wird die Optik 52 der Vorrichtung 1 zumindest teilweise in die Bohrung 62 eingeführt. Die Optik 52 muss nicht im Zentrum der Bohrung 62 angeordnet sein. Eventuelle Zentrierungsfehler können dadurch ausgeglichen werden, dass zwei Beleuchtungsstreifen 19 auf einander gegenüberliegende Bereiche der Innenwand 61 der Bohrung 62 treffen. Die Optik 52 bzw. die Vorrichtung 1 wird im Inneren der Bohrung 62 gedreht, so dass die Bohrung 62 über ihren gesamten Umfang untersucht werden kann.

Gemäß Figur 7 umfasst die Optik 52 der Vorrichtung 1 zusätzlich zu den Bauteilen gemäß Figur 5 die zwei Strahlteiler 63. Die Optik 52 gemäß Figur 7 ist derart ausgebildet, dass sie in Bohrung 62 mit einem Durchmesser von beispielsweise 11 mm bis 300 mm einführbar ist.

Figur 8 zeigt die erfindungsgemäße Vorrichtung 1 gemäß einer siebten Ausführungsform, die auf dem Prinzip des Michelson-Aufbaus (Figur 2) beruht. Im Vergleich zur Vorrichtung 1 gemäß Figur 5 ist in dem mikro-optischen Array 38 ein Hologramm integriert, mit dem zwei Strahlenbündel 64 gebildet werden, die als Beleuchtungsstreifen 19 auf das Objekt fallen. Die Strahlenbündel 64 werden über die Linsen 14, 53, 56 und den Spiegel 57 auf das Objekt 15 gelenkt.

Weiter teilt das mikro-optische Array 38 der Vorrichtung 1 im Gegensatz zu dem in Figur 5 Gezeigten die Laserstrahlung der Laser-Emitter 2 in die Referenzstrahlung 5 und die Beleuchtungsstrahlung 6 auf.

Darüber hinaus wird kein Rückreflektor 54 (vgl. Figur 5) in die Linse 53 integriert, sondern ein Parabolspiegel 10 verwendet, um die Referenzstrahlung 5 vom Laser-Emitter 2 zum Detektor 12 zu lenken. Die aus dem mikro-optischen Array 38 austretende Referenzstrahlung 5 wird am Parabolspiegel 10 reflektiert und dann an dem zweiten Strahlteiler 13 derart reflektiert, dass sie auf einen Analysator (Polarisator) 65 und danach auf den Detektor 12 trifft. Der Parabolspiegel 10 ist derart ausgebildet, dass die Referenzstrahlung 5 je nach Position des Laser-Emitters 2 auf dem Emitter-Chip 3 unter einem bestimmten Referenzeinfallswinkel α (vgl. Figur 1a) auf dem Detektor 12 auftrifft.

Referenzstrahlung 5 und Beleuchtungsstrahlung 6 verlassen das mikro-optische Array 38 mit unterschiedlichen Polarisationszuständen. Der zweite Strahlteiler 13 ist als polarisationssensitiver Strahlteiler ausgebildet, so dass er die Referenzstrahlung 5 mit einer ersten Polarisation durchlässt und die Beleuchtungsstrahlung 6 mit einer zweiten Polarisation reflektiert. Dies ist natürlich auch umgekehrt denkbar.

Die zweite Linse 53 weist auf ihrer Vorderseite 55 einen Zirkulator (nicht dargestellt) auf, der die Beleuchtungsstrahlung 6 und danach die vom Objekt 15 reflektierte Objektstrahlung 21 (vgl. Figur 5) um insgesamt 90° dreht, so dass sie in Richtung Detektor 12 durch den zweiten Strahlteiler 13 transmittiert wird und auf den Detektor 12 fällt.

Der Analysator (Polarisator) 65 stellt sicher, dass das Interferenzmuster durch identische Polarisationsebenen aus dem Objektstrahl 21 und dem Referenzstrahl 5 gebildet werden kann.

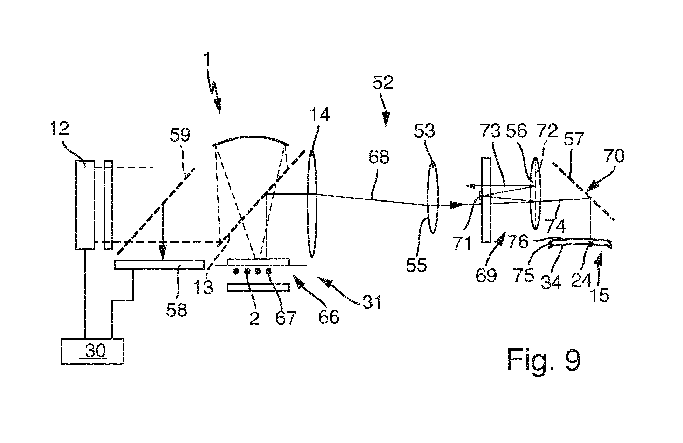

Figur 9 zeigt die erfindungsgemäße Vorrichtung 1 gemäß einer achten Ausführungsform, die sich von der Ausführungsform gemäß Figur 8 durch eine Messeinheit 31 zur Messung der Dicke einer auf dem Objekt 15 angeordneten Schicht 75 unterscheidet. Die Messeinheit 31 weist einen Weißlichtpunktsensor 66 auf, der nach dem bekannten Prinzip der Interferometrie arbeitet.

Der Weißlichtpunktsensor 66 umfasst als Lichtquelle 67 eine Punktlichtquelle mit breitem Spektrum, bevorzugt mit einer Breite des Spektrums von 10 nm bis 100 nm. Die Lichtquelle 67 strahlt um eine zentrale Wellenlänge von bevorzugt 1300 nm, welche in streuenden Medien (z.B. Gewebe besonders transparent ist). Die Lichtstrahlung der Lichtquelle 67 ist beispielhaft durch den Lichtstrahl 68 dargestellt.

Teil des Weißlichtpunktsensors 66 ist weiter ein zweiter Detektor 58, der die Lichtstrahlung der Lichtquelle 67 detektiert. Um die Lichtstrahlung zu diesem zweiten Detektor 58 zu lenken, weist die Vorrichtung 1 einen zusätzlichen Strahlteiler 59 auf, der in Abhängigkeit von der Wellenlänge Strahlung reflektiert oder transmittiert.

Für die Schichtdickenmessung basierend auf dem Verfahren der Interferometrie umfasst die Vorrichtung 1 weiter einen zweiten Referenzarm 69 und einen zweiter Objektarm 70. Der zweite Referenzarm 69 ist definiert als der doppelte Abstand zwischen einem in der Optik 52 der Vorrichtung 1 angeordnetem Referenzspiegel 71 und einem halbdurchlässigen Spiegel 72 in der dritten Linse 56. Der zweite Objektarm 70 ist definiert als der Abstand zwischen dem halbdurchlässigen Spiegel 72 und einem Objektpunkt 24 des Objekts 15. Referenzarm 69 und Objektarm 70 sind bis auf Wegstreckendifferenzen von 0,1 mm bis maximal z.B. 3 mm bewusst nicht ganz identisch, vorausgesetzt, die Vorrichtung 1 befindet sich in einem entsprechenden Abstand zum Objekt 15.

Im Folgenden wird der optische Pfad des Weißlichtpunktsensors 66 erläutert. Für seine Funktion ist nach dem Stand der Technik bekannt, dass mittels durchstimmbarer Wellenlänge die Schichtdickeninformation über die Frequenzmessung an einem Detektor 58 erfolgen kann. Dazu muss die Wegstrecken-Differenz zwischen Objektarm 70 und Referenzarm 69 von Null verschieden sein.

Die Lichtquelle 67 des Weißlichtpunktsensors 66 sendet einen Lichtstrahl 68 aus, der über den Strahlteiler 13, durch die Linse 14 und die zweite Linse 53 am Referenzspiegel 71 vorbei auf den halbdurchlässigen Spiegel 72 gelenkt wird. Der halbdurchlässigen Spiegel 72 teilt den Lichtstrahl 68 in einen Referenzstrahl 73, der reflektiert wird, und einen Beleuchtungsstrahl 74, der durchgelassen wird, auf.

Der am halbdurchlässigen Spiegel 72 reflektierte Referenzstrahl 73 trifft auf den Referenzspiegel 71, wird von diesem auf den halbdurchlässigen Spiegel 72 zurückgeworfen und in Richtung des zweiten Strahlteilers 13 erneut reflektiert. Es ist auch denkbar, den Referenzspeiegel 71 als Hologramm auszubilden, das den Referenzstrahl 73 auf den für die Wellenlänge des WeißlichtPunktsensors 66 sensitiven halbdurchlässigen Spiegel 72 zurückwirft.

Der vom halbdurchlässigen Spiegel 72 transmittierte Beleuchtungsstrahl 74 wird über den Spiegel 57 auf das Objekt 15 abgelenkt. Der Beleuchtungsstrahl 74 durchdringt eine auf dem Objekt 15 befindliche Schicht 75 und trifft u.a. auf den Objektpunkt 24 des Objekts 15. Der Verlauf des am Objektpunkt 24 als Objektstrahl (nicht dargestellt) reflektierten Beleuchtungsstrahls 74 erfolgt analog zu der am Objekt 15 in Figur 8 reflektierten Objektstrahlung 21 Allerdings wird der Objektstrahl gemäß der achten Ausführungsform der Vorrichtung 1 aufgrund seiner Wellenlänge vom zusätzlichen Strahlteiler 59 reflektiert und auf den Detektor 58 gelenkt.

Mithilfe der von den Laser-Emittern 2 ausgesendeten Referenzstrahlung 5 und Beleuchtungsstrahlung 6 (vgl. Figur 8) ist die Vorrichtung 1 analog zu der Vorrichtung 1 aus Figur 8 in der Lage, eine Oberfläche 76 der Schicht 75 zu erfassen. Die Messeinheit 31 ermöglicht zusätzlich das Objekt 15 unterhalb der Schicht 75 abzutasten. Da sowohl die Oberfläche 76 wie auch das Objekt 15 unterhalb der Schicht 75 ermittelbar ist, kann mithilfe der Verarbeitungseinrichtung 30 die Schichtdicke der Schicht 75 berechnet werden.

Die in den Figuren 5 bis 9 dargestellten Ausführungsformen der Vorrichtung 1 können als Dentalscanner, beispielsweise zum dreidimensionalen Erfassen eines Zahns oder ganzen Kiefers eingesetzt werden. Hierfür weist die Optik 52 bevorzugt einen Durchmesser von höchstens 20 mm, bevorzugt von höchstens 10 mm auf. Die Optik 52 hat eine Länge von höchstens 150 mm, bevorzugt von höchstens 100 mm. Die Länge der Optik 52 ist definiert als der Abstand zwischen der Linse 14 und dem objektseitigen Ende der Vorrichtung 1.

## Patentansprüche

1. Vorrichtung zum Erfassen einer 3D-Struktur eines Objekts (15) umfassend:
- einen ersten Laser-Emitter (2a), der Laserstrahlung mit einer ersten Wellenlänge erzeugt,
- einen zweiten Laser-Emitter (2b), der Laserstrahlung mit einer zweiten Wellenlänge erzeugt,
wobei sich die erste Wellenlänge von der zweiten Wellenlänge unterscheidet,
- optische Einrichtungen (4, 9, 10, 13, 14, 38, 50, 53, 56), von denen wenigstens eine ein Strahlteiler (4, 48, 50, 54) ist, der die Laserstrahlung der Laser-Emitter (2, 2a, 2b) jeweils in eine Referenzstrahlung (5) und eine Beleuchtungsstrahlung (6) aufteilt, wobei die Beleuchtungsstrahlung (6) auf das zu vermessende Objekt (15) trifft, von dem Objekt (15) als Objektstrahlung (21) reflektiert wird und mit der Referenzstrahlung (5) interferiert
- einen Detektor (12), der die daraus entstehenden Interferenzmuster aufnimmt und
- eine Messeinrichtung (27), welche es erlaubt, den zeitlichen Verlauf der Wellenlängen der Laserstrahlung der Laser-Emitter (2, 2a, 2b) zu messen,
**dadurch gekennzeichnet,**
**dass** die Laser-Emitter (2, 2a, 2b) derart angeordnet sind, dass die Beleuchtungsstrahlung (6) des ersten Laser-Emitters (2a) und die Beleuchtungsstrahlung (6) des zweiten Laser-Emitters (2b) mit unterschiedlichen Einfallswinkeln (β) auf das Objekt (15) treffen und,
**dass** die Messeinrichtung mit einer Steuereinrichtung (28) verbunden ist, die dazu geeignet ist, den Detektor (12) im Falle im Wesentlichen konstanter Wellenlängen anzusteuern und eine Aufnahme der Interferenzmuster auszulösen.

2. Vorrichtung nach Anspruch 1, **dadurch gekennzeichnet, dass** die Vorrichtung (1) eine Regeleinrichtung (29) aufweist, die in Abhängigkeit der Messergebnisse der Messeinrichtung (27) die Laser-Emitter (2, 2a, 2b) derart regelt, dass die Wellenlängen der emittierten Laserstrahlung im Wesentlichen konstant sind.

3. Vorrichtung nach Anspruch 1 oder 2, **dadurch gekennzeichnet, dass** die Messeinrichtung als Fabry Perot Interferometer (27) ausgebildet ist.

4. Vorrichtung nach einem der vorhergehenden Ansprüche, **dadurch gekennzeichnet, dass** der erste Laser-Emitter (2a) und der zweite Laser-Emitter (2b) auf einem gemeinsamen Emitter-Chip (3) voneinander beabstandet angeordnet sind.

5. Vorrichtung nach einem der vorhergehenden Ansprüche, **dadurch gekennzeichnet, dass** wenigstens eine der optischen Einrichtungen (4, 9, 10, 13, 54) ausgebildet ist, die Referenzstrahlung (5) derart zu reflektieren, dass die Referenzstrahlung (5) der einzelnen Laser-Emitter (2) mit unterschiedlichen Referenzeinfallswinkeln (α) auf den Detektor (12) treffen.

6. Vorrichtung nach einem der vorhergehenden Ansprüche, **dadurch gekennzeichnet, dass** wenigstens eine der optischen Einrichtungen ein Hologramm (50) ist, das die Beleuchtungsstrahlung (5) derart ablenkt, dass sie als ein oder mehrere Beleuchtungsstreifen (19) auf das Objekt (15) trifft

7. Vorrichtung nach einem der vorherigen Ansprüche, **dadurch gekennzeichnet, dass** ein mikro-optisches Array (38) den Strahlteiler (48, 50) umfasst, der die Laserstrahlung der Laser-Emitter (2, 2a, 2b) in die Referenzstrahlung (5) und die Beleuchtungsstrahlung (6) aufteilt und beide Strahlungen (5, 6) mit unterschiedlichen Strahlungsprofilen beaufschlagt

8. Vorrichtung nach Anspruch 7, **dadurch gekennzeichnet, dass** das mikro-optische-Array (38) mindestens einen Polarisator (47, 51) umfasst, um die Beleuchtungsstrahlung (6) und/oder die Referenzstrahlung (5) der Laser-Emitter (2, 2a, 2b) zu polarisieren.

9. Vorrichtung nach Anspruch 7 , **dadurch gekennzeichnet, dass** das mikro-optische Array (38) mindestens ein Hologramm (50) umfasst.

10. Vorrichtung nach einem der vorhergehenden Ansprüche, **dadurch gekennzeichnet, dass** wenigstens eine der optischen Einrichtungen als chromatisch dispersive Linse (14, 53, 56) oder als chromatisch dispersiver Spiegel (10) ausgebildet ist.

11. Vorrichtung nach einem der vorhergehenden Ansprüche **dadurch gekennzeichnet, dass** die Vorrichtung (1) zwei Emitter-Chips (3) und zwei Detektoren (12, 58) aufweist, wobei die Laserstrahlung der auf dem einen Emitter-Chip (3) angeordneten Laser-Emitter (2, 2a, 2b) auf den einen Detektor (12) und die Laserstrahlung der auf dem anderen Emitter-Chip (3) angeordneten Laser-Emitter (2, 2a, 2b) auf den anderen Detektor (58) trifft.

12. Vorrichtung nach einem der Ansprüche 1 bis 11, **dadurch gekennzeichnet, dass** die Vorrichtung (1) eine Messeinheit (31) zur Messung der Dicke einer auf dem Objekt (15) angeordneten Schicht (75) aufweist.

13. Vorrichtung nach Anspruch 12, **dadurch gekennzeichnet, dass** die Messeinheit (31) einen Weißlicht-Punktsensor (66) umfasst, der nach dem Prinzip der frequenz-scannenden Interferometrie arbeitet.

14. Vorrichtung nach Anspruch 12, **dadurch gekennzeichnet, dass** die Messeinheit (31) ausgebildet ist, basierend auf dem Prinzip der Ellipsometrie die Dicke der auf dem Objekt (15) angeordneten Schicht (75) zu ermitteln.

15. Vorrichtung nach einem der vorhergehenden Ansprüche, **dadurch gekennzeichnet, dass** die Vorrichtung (1) eine Optik (52) mit mehreren optischen Einrichtungen (50, 53, 56, 57, 63) aufweist, wobei die Optik (52) derart ausgebildet ist, dass sie in den Mundinnenbereich eines Patienten oder in eine Bohrung (62) einführbar ist.

## Claims

1. Apparatus for detecting a 3D structure of an object (15) comprising:
- a first laser emitter (2a), which generates laser radiation having a first wavelength,
- a second laser emitter (2b), which generates laser radiation having a second wavelength,
wherein the first wavelength differs from the second wavelength,
- optical devices (4, 9, 10, 13, 14, 38, 50, 53, 56), of which at least one is a beam splitter (4, 48, 50, 54), which splits the laser radiation of the laser emitters (2, 2a, 2b) in each case into a reference radiation (5) and an illuminating radiation (6), wherein the illuminating radiation (6) impinges upon the object (15) to be measured, is reflected by the object (15) as object radiation (21) and interferes with the reference radiation (5),
- a detector (12), which records the interference patterns formed therefrom, and
- a measuring device (27) allowing to measure the time behaviour of the wavelengths of the laser radiation of the laser emitters (2, 2a, 2b)
**characterized in,**
**that** the laser emitters (2, 2a, 2b) are located in such a manner that the illuminating radiation (6) of the first laser emitter (2a) and the illuminating radiation (6) of the second laser emitter (2b) impinge upon the object (15) at different angles of incidence (β) and
**that** the measuring device (27) is connected to a control device (28) which is capable of actuating the detector (12) and triggering a recording of the interference patterns in the case of substantially constant wavelengths.

2. The apparatus according to claim 1, **characterized in that** the apparatus (1) comprises a regulating device (29), which depending on the measurement results of the measuring device (27), regulates the laser emitters (2, 2a, 2b) in such a manner that the wavelengths of the emitted laser radiation are substantially constant.

3. The apparatus according to claim 1 or 2, **characterized in that** the measuring device is configured as a Fabry Perot interferometer (27).

4. The apparatus according to any one of the preceding claims, **characterized in that** the first laser emitter (2a) and the second laser emitter (2b) are located on a common emitter chip (3) spaced apart from one another.

5. The apparatus according to any one of the preceding claims, **characterized in that** at least one of the optical devices (4, 9, 10, 13, 54) is configured to reflect the reference radiation (5) in such a manner that the reference radiation (5) of the individual laser emitters (2) is incident on the detector (12) at different reference angles of incidence (α).

6. The apparatus according to any one of the preceding claims, **characterized in that** at least one of the optical devices is a hologram (50) which deflects the illuminating radiation (5) in such a manner that it impinges upon the object (15) as one or more illuminating strips (19).

7. The apparatus according to any one of the preceding claims, **characterized in that** a micro-optic array (38) comprises the beam splitter (48, 50) which splits the laser radiation of the laser emitters (2, 2a, 2b) into the reference radiation (5) and the illuminating radiation (6) and supplies both sets of radiation (5, 6) with different radiation profiles.

8. The apparatus according to claim 7, **characterized in that** the micro-optic array (38) comprises at least one polarizer (47, 51) in order to polarize the illuminating radiation (6) and/or the reference radiation (5) of the laser emitters (2, 2a, 2b).

9. The apparatus according to claim 7, **characterized in that** the micro-optic array (38) comprises at least one hologram (50).

10. The apparatus according to any one of the preceding claims, **characterized in that** at least one of the optical devices is configured as a chromatically dispersive lens (14, 53, 56) or as a chromatically dispersive mirror (10).

11. The apparatus according to any one of the preceding claims, **characterized in that** the apparatus (1) comprises two emitter chips (3) and two detectors (12, 58), wherein the laser radiation of the laser emitters (2, 2a, 2b) located on the one emitter chip (3) impinges upon the one detector (12) and the laser radiation of the laser emitters (2, 2a, 2b) located on the other emitter chip (3) impinges upon the other detector (58).

12. The apparatus according to any one of claims 1 to 11, **characterized in that** the apparatus (1) comprises a measuring unit (31) for measuring the thickness of a layer (75) located on the object (15).

13. The apparatus according to claim 12, **characterized in that** the measuring unit (31) comprises a white light point sensor (66), which operates according to the principle of frequency-scanning interferometry,

14. The apparatus according to claim 12, **characterized in that** the measuring unit (31) is configured to determine the thickness of the layer (75) located on the object (15) based on the principle of ellipsometry.

15. The apparatus according to any one of the preceding claims, **characterized in that** the apparatus (1) comprises optics (52) having a plurality of optical devices (50, 53, 56, 57, 63), wherein the optics (52) are configured in such a manner that it can be inserted into the inner mouth region of a patient or into a bore (62).

## Revendications

1. Dispositif d'enregistrement d'une structure 3D d'un objet (15), comprenant
- un premier émetteur laser (2a), qui produit un rayonnement laser avec une première longueur d'onde,
- un deuxième émetteur laser (2b), qui produit un rayonnement laser avec une deuxième longueur d'onde,
dans lequel la première longueur d'onde diffère de la deuxième longueur d'onde,
- des dispositifs optiques (4, 9, 10, 13, 14, 38, 50, 53, 56), dont au moins un est un diviseur de faisceau (4, 48, 50, 54), qui divise le rayonnement laser des émetteurs laser (2, 2a, 2b) chaque fois en un rayonnement de référence (5) et un rayonnement d'éclairage (6), dans lequel le rayonnement d'éclairage (6) arrive sur l'objet à mesurer (15), est réfléchi par l'objet (15) sous forme de rayonnement d'objet (21) et interfère avec le rayonnement de référence (5),
- un détecteur (12), qui enregistre les motifs d'interférence qui en résultent, et
- un dispositif de mesure (27), qui permet de mesurer l'évolution temporelle des longueurs d'onde du rayonnement laser des émetteurs laser (2, 2a, 2b),
**caractérisé en ce que**
les émetteurs laser (2, 2a, 2b) sont disposés de telle manière que le rayonnement d'éclairage (6) du premier émetteur laser (2a) et le rayonnement d'éclairage (6) du deuxième émetteur laser (2b) arrivent sur l'objet (15) sous des angles d'incidence différents (3), et
le dispositif de mesure est relié à un dispositif de commande (28), qui est apte à commander le détecteur (12) dans le cas de longueurs d'onde essentiellement constantes et de déclencher un enregistrement des motifs d'interférence.

2. Dispositif selon la revendication 1, **caractérisé en ce que** le dispositif (1) présente un dispositif de régulation (29), qui régule les émetteurs laser (2, 2a, 2b) en fonction des résultats de mesure du dispositif de mesure (27), de telle manière que les longueurs d'onde du rayonnement laser émis soient essentiellement constantes.

3. Dispositif selon la revendication 1 ou 2, **caractérisé en ce que** le dispositif de mesure est constitué par un interféromètre de Fabry-Pérot (27).

4. Dispositif selon l'une quelconque des revendications précédentes, **caractérisé en ce que** le premier émetteur laser (2a) et le deuxième émetteur laser (2b) sont disposés sur une puce d'émetteur commune (3) à distance l'un de l'autre.

5. Dispositif selon l'une quelconque des revendications précédentes, **caractérisé en ce qu'**au moins un des dispositifs optiques (4, 9, 10, 13, 54) est configuré de façon à réfléchir le rayonnement de référence (5) d'une manière telle que le rayonnement de référence (5) des émetteurs laser individuels (2) arrive sur le détecteur (12) sous des angles d'incidence de référence (α) différents.

6. Dispositif selon l'une quelconque des revendications précédentes, **caractérisé en ce qu'**au moins un des dispositifs optiques est un hologramme (50), qui dévie le rayonnement d'éclairage (5) d'une manière telle qu'il arrive sur l'objet (15) sous la forme d'une ou de plusieurs bandes d'éclairage (19).

7. Dispositif selon l'une quelconque des revendications précédentes, **caractérisé en ce qu'**un réseau micro-optique (38) comprend le diviseur de faisceau (48, 50), qui divise le rayonnement laser des émetteurs laser (2, 2a, 2b) en un rayonnement de référence (5) et un rayonnement d'éclairage (6) et dote les deux rayonnements (5, 6) de profils de rayonnement différents.

8. Dispositif selon la revendication 7, **caractérisé en ce que** le réseau micro-optique (38) comprend au moins un polariseur (47, 51), afin de polariser le rayonnement d'éclairage (6) et/ou le rayonnement de référence (5) des émetteurs laser (2, 2a, 2b).

9. Dispositif selon la revendication 7, **caractérisé en ce que** le réseau micro-optique (38) comprend au moins un hologramme (50).

10. Dispositif selon l'une quelconque des revendications précédentes, **caractérisé en ce qu'**au moins un des dispositifs optiques est constitué par une lentille à dispersion chromatique (14, 53, 56) ou un miroir à dispersion chromatique (10).

11. Dispositif selon l'une quelconque des revendications précédentes, **caractérisé en ce que** le dispositif (1) présente deux puces d'émetteur (3) et deux détecteurs (12, 58), dans lequel le rayonnement laser des émetteurs laser (2, 2a, 2b) disposés sur une première puce d'émetteur (3) arrive sur un premier détecteur (12) et le rayonnement laser des émetteurs laser (2, 2a, 2b) disposés sur l'autre puce d'émetteur (3) arrive sur l'autre détecteur (58).

12. Dispositif selon l'une quelconque des revendications 1 à 11, **caractérisé en ce que** le dispositif (1) présente une unité de mesure (31) pour la mesure de l'épaisseur d'une couche (75) disposée sur l'objet (15).

13. Dispositif selon la revendication 12, **caractérisé en ce que** l'unité de mesure (31) comprend un détecteur de point à lumière blanche (66), qui travaille selon le principe de l'interférométrie à balayage de fréquence.

14. Dispositif selon la revendication 12, **caractérisé en ce que** l'unité de mesure (31) est configurée de façon à déterminer l'épaisseur de la couche (75) disposée sur l'objet (15) en se basant sur le principe de l'ellipsométrie.

15. Dispositif selon l'une quelconque des revendications précédentes, **caractérisé en ce que** le dispositif (1) présente une optique (52) avec plusieurs dispositifs optiques (50, 53, 56, 57, 63), dans lequel l'optique (52) est configurée de telle manière qu'elle puisse être introduite dans la cavité buccale d'un patient ou dans un alésage (62)
